(19) Europäisches Patentamt / European Patent Office / Office européen des brevets

(11) **EP 1 474 529 B1**

(12) **EUROPEAN PATENT SPECIFICATION**

(45) Date of publication and mention
of the grant of the patent:
**22.10.2008 Bulletin 2008/43**

(51) Int Cl.:
**C12Q 1/68** (2006.01)

(21) Application number: **03704579.6**

(22) Date of filing: **10.02.2003**

(86) International application number:
**PCT/EP2003/001276**

(87) International publication number:
**WO 2003/066897 (14.08.2003 Gazette 2003/33)**

(54) **SPECIFIC MULTIPLEX ANALYSIS OF NUCLEIC ACIDS**

SPEZIFISCHE MULTIPLEX-ANALYSE VON NUKLEINSÄUREN

ANALYSE MULTIPLEX SPECIFIQUE D'ACIDES NUCLEIQUES

(84) Designated Contracting States:
**AT BE BG CH CY CZ DE DK EE ES FI FR GB GR HU IE IT LI LU MC NL PT SE SI SK TR**

(30) Priority: **08.02.2002 US 354518 P**
**08.02.2002 EP 02002854**

(43) Date of publication of application:
**10.11.2004 Bulletin 2004/46**

(73) Proprietor: **Olympus Corporation**
**Shibuya-ku, Tokyo (JP)**

(72) Inventors:
• **ZIRWES, Rudolf**
**41352 Korschenbroich (DE)**
• **KOPPERS, Elke**
**47669 Wachtendonk (DE)**
• **GORES, Imke**
**45239 Essen (DE)**

(74) Representative: **Meyers, Hans-Wilhelm**
**Patentanwälte**
**von Kreisler-Selting-Werner**
**Postfach 10 22 41**
**50462 Köln (DE)**

(56) References cited:
**EP-A- 0 332 435**     **EP-A- 1 061 135**

• BOLDT B ET AL: "ONE-TUBE METHOD FOR COMPLETE HPA-1 GENOTYPING BY PCR USING SEQUENCE-SPECIFIC PRIMERS" BRITISH JOURNAL OF HAEMATOLOGY, OXFORD, GB, vol. 99, no. 4, December 1997 (1997-12), pages 968-973, XP001096153 ISSN: 0007-1048
• HAFF L A: "IMPROVED QUANTITATIVE PCR USING NESTED PRIMERS" PCR METHODS & APPLICATIONS, COLD SPRING HARBOR LABORATORY PRESS, US, vol. 3, no. 6, 1 June 1994 (1994-06-01), pages 332-337, XP000561192 ISSN: 1054-9803 cited in the application
• KWOK S ET AL: "Effects of primer-template mismatches on the polymerase chain reaction: human immunodeficiency virus type 1 model studies" NUCLEIC ACIDS RESEARCH, OXFORD UNIVERSITY PRESS, SURREY, GB, vol. 18, no. 4, 25 February 1990 (1990-02-25), pages 999-1005, XP002220845 ISSN: 0305-1048
• NEWTON ET AL: "Analysis of any point mutation in DNA. The amplification refractory mutation system (ARMS)." NUCLEIC ACID RESEARCH, vol. 17, no. 7, - 1989 pages 2503-2516,
• VON AHSEN ET AL: CLINICAL CHEMISTRY, vol. 45, no. 12, - 1999 pages 2094-2101,

SEQUENCE LISTING

<110> Evotec Technologies GmbH

<120> Specific multiplex analysis of nucleic acids

<130> 023500wo ME/BM

<140> PCT/EP03/01276
<141> 2003-02-10

<160> 10

<170> PatentIn Ver. 2.1

<210> 1
<211> 24
<212> DNA
<213> Artificial Sequence

<220>
<223> Description of Artificial Sequence: forward amplification primer (F) for hMLH1

<400> 1
ctgatgttag gacactaccc aatg      24

<210> 2
<211> 21
<212> DNA
<213> Artificial Sequence

<220>
<223> Description of Artificial Sequence: reverse amplification primer (R) for hMLH1

<400> 2
agaaggagca ttggaggata a      21

<210> 3
<211> 11
<212> DNA
<213> Artificial Sequence

<220>
<223> Description of Artificial Sequence: wildtype-specific diagnostic primer (D1) for hMLH1

<400> 3
gctccatctt t      11

<210> 4
<211> 11
<212> DNA
<213> Artificial Sequence

<220>
<223> Description of Artificial Sequence: mutant-specific diagnostic primer (D2) for hMLH1

<400> 4
gctccgtctt t      11

<210> 5
<211> 33
<212> DNA
<213> Artificial Sequence

<220>
<223> Description of Artificial Sequence: wildtype-specific chimeric primer (CD1) for hMLH1

<400> 5
aggtcactcg cacagatgtc gctccatctt tgg      33

<210> 6
<211> 31
<212> DNA
<213> Artificial Sequence

<220>
<223> Description of Artificial Sequence: mutant-specific chimeric primer (CD2) for hMLH1

<400> 6
aggtaaccac gctcaatgcg ctccgtcttt g      31

<210> 7
<211> 18
<212> DNA
<213> Artificial Sequence

<220>
<223> Description of Artificial Sequence: 5'-tail-specific labeling primer (L1) for CD1

<400> 7
aggtcactcg cacagatg          18

<210> 8
<211> 18
<212> DNA
<213> Artificial Sequence

<220>
<223> Description of Artificial Sequence: 5'-tail-specific labeling primer (L2) for CD2

<400> 8
aggtaaccac gctcaatg          18

<210> 9
<211> 20
<212> DNA
<213> Artificial Sequence

<220>
<223> Description of Artificial Sequence: sense primer for hMHL1 genotyping

<400> 9
gtttgctggt ggagataagg          20

<210> 10
<211> 39
<212> DNA
<213> Artificial Sequence

<220>
<223> Description of Artificial Sequence: antisense primer for hMHL1 genotyping

<400> 10
ataggttatc gacataccga ctaacagcat ttccatcga          39

**Description**

The present invention provides a novel type of amplification and detection method that allows homogeneous multiplex analysis of nucleic acid sequences and the detection of single nucleotide polymorphisms and nucleotide sequence changes such as point-mutations, variant alleles, deletions, insertions, repeats and/or inversions. Additionally, the method of the present invention enables assaying the cytosine methylation status. Moreover, the present invention may be useful for the quantification of nucleic acids and for genotyping.

Homogeneous assays for the detection of nucleic acid sequences, especially for the detection of single-nucleotide polymorphisms (SNPs), are an important tool for genetic analysis and have been developed into commercial products particularly for clinical diagnostics. SNPs are evolutionary stable point mutations scattered throughout the human genome at every 500 to 1000 DNA bases and, therefore, represent the most common genetic variation in human populations (Cooper et al., Hum Genet 1985, 69:201-205; Collins et al., Genome Res 1998, 8:1229-1231). The genome of any two non-related individuals is estimated to differ in at least three million nucleotide positions, of which approximately 500,000 are located in coding regions. The availability of the draft sequence of the complete human genome (Lander et al., Nature 2001, 409:860-921; Venter et al., Science 2001, 291:1304-1351) now provides the basis for the generation of a whole-genome SNP map, a powerful tool for genetic analysis which will uncover the association of SNPs with many disease traits, and holds great promise for the optimization of new drug development and the individualization of clinical diagnostics and therapeutics (Bentley, Med Res Rev 2000, 20:189-196; Pfost et al., Trends Biotechnol 2000, 18:334-338; Schork et al., Clin Genet 2000, 58:250-264). Future developments will focus on identifying and/or screening SNPs relevant to certain disease areas (e.g. cancer, neurodegenerative or cardiovascular disease) in large patient collectives and on performing whole-genome scans of SNPs in individuals. Methods fufilling the requirements of these diverse developments must allow flexible and rapid assays, medium- to high-throughput and high-quality genotyping for SNPs of interest. Currently applied methods for the analysis of nucleic acid sequences, especially the analysis of SNPs, include restriction fragment-length polymorphism analysis of polymerase chain redaction products-(RFLP-PCR), hybridization with allele-specific oligonucleotides (Nickerson et al., Proc Natl Acad Sci USA 1990, 87:8923-8927; Saiki et al., Proc Natl Acad- Sci USA 1989, 86:6230-6234; de Verlaan et al., Gene 1986, 50:313-320; Wallace et al, Nucleic Acids Res 1981, 9:879-894; Zhang et al., Nucleic Acids Res 1991, 19:3929-3933), allele-specific PCR (Gibbs et al., Nucleic Acids Res 1989, 17:2437-2448; Newton et al., Nucleic Acids Res 1989, 17:2503-2516), oligonucleotide ligation assay (Grossman et al., Nucleic Acids Res 1994, 22:4527-4534; Landegren et al., Science 1988, 241:1077-1080), allele-specific ligase chain reaction (Abravaya et al., Nucleic Acids Res 1995, 23:675-682; Barany, Proc Natl Acad Sci USA 1991, 88: 189-193; Wu und Wallace, Genomics 1989,4:560-569), high-density chip arrays for allele-specific hybridization analysis (Wang et al., Science 1998, 280:1077-1082; Yershov et al., Proc Natl Acad Sci USA 1996, 93:4913-4918), the 5'-nuclease assay (Holland et al., Proc Natl Acad Sci USA 1991, 88:7276-7280; Lee et al., Biotechniques 1999, 27:342-349; Livak, Genet Anal 1999, 14:143-149; Livak et al., PCR Methods Appl 1995, 4:357-362), the template-directed dye-terminator incorporation assay (Chen and Kwok, Nucleic Acids Res 1997, 25:347-353; Chen et al., Genome Res 1999, 9:492-498 and Proc Natl. Acad Sci USA 1997, 94:10756-10761), the molecular beacon allele-specific oligonucleotide assay (Marras et al., Genet Anal 1999, 14:151-156; Tyagi and Kramer, Nat Biotechnol 1996, 14:303-308), the allele-specific fluorescence energy transfer (LightCycler) assay (Bernard et al., Anal Biochem 1998, 255:101-107; Lay and Wittwer, Clin Chem 1997, 43:2262-2267), the structure-specific invasive endonuclease cleavage assay (Lyamichev et al., Nat Biotechnol 1999, 17:292-296), and the pyrosequencing assay (Ahmadian et al., Anal Biochem 2000, 280:103-110, Alderborn et al., Genome Res 2000, 10:1249-1258).

Kwok S. et al (Nucleic Acids Research, vol. 18, no. 4, 999-1005, 1990) investigated the effects of various primer-template mismatches on DNA amplification of an HIV-1 *gag* region by the polymerase chain reaction (PCR).

Most of the methods currently employ macroscopic fluorescence techniques, encompassing conventional fluorescence intensity, fluorescence polarization, energy transfer, or fluorescence quenching, which monitor the average fluorescence output from the ensemble of emitting fluorophores. In contrast, the single-molecule detection technology fluorescence correlation spectroscopy (FCS) (Eigen and Rigler, *Proc Natl* Acad Sci USA 1994, 91:5740-5747; Elson and Magde, Biopolymers 1974, 13:1-27, Magde et al., Phys Rev Lett 1972, 29:705-708 and Biopolymers 1974, 13:29-61) statistically samples the fluorescence fluctuations of single molecules in discrete time intervals with a temporal resolution down to nanoseconds and below. Many such detection events are then averaged to define the properties of the whole assay system. Analysis of single molecular events is accomplished using confocal optics with an illumination/observation volume of approximately 0.24 fl ($10^{-15}$ liter) and has several advantages over conventional macroscopic fluorescence methods. For example, problems derived from probe adsorption, autofluorescence of reaction components or of reaction vessels, as well as inner filter effects are virtually eliminated. This results in a high signal-to-noise ratio and maximum sensitivity which is essentially independent of the assay volume and allows ultimate assay miniaturization down to 1 $\mu$l or below, an essential prerequisite of high-throughput analysis. Autocorrelation of fluorescence fluctuations provides multiple fluoresence parameters including the average numbers and translational diffusion times of fluorescent molecules in the confocal illumination/observation volume. FCS has been successfully applied to monitor molecular interactions as diverse as drug/target-interactions (Auer et al., Drug Discovery Today 1998, 3:457-465; Sterrer and Henco, J Recept Signal Transduct Res 1997, 17:511-520), enzymatic and binding studies (Kettling et al., Proc Natl Acad Sci USA 1998, 95:1416-1420; Meyer-Almes and Auer, Biochemistry 2000, 39:13261-13268) as well as protein aggregation studies (Pitschke et al., Nat Med 1998, 4:832-834, Tjernberg et al., Chem Biol 1999, 6:53-62). In addition, FCS has been shown to be well-suited for qualitative and quantitative nucleic acid analysis (Bjorling et al., Biochemistry 1998, 37:12971-12978; Kinjo, BioTechniques 1998, 25:705-6; Kinjo et al., Anal Biochem 1998, 260:166-172; Kinjo and Rigler, Nucleic Acids Res 1995, 23:1795-1799; Rigler et al., J Biotechnol 1998, 63:97-109; Walter et al., Proc Natl Acad Sci USA 1996, 93: 12805-12810; Weiner et al., Digestion 2000, 61:84-89) and for indirect detection of point mutations in genes (Kinjo and Nishimura, Bioimaging 1997, 5:134-138).

European Patent No. 332435 ("Method of detecting nucleotide sequences") of Zeneca Ltd., London, and Newton et al. (Nucleic Acid Res 1989, 17:2503-16) describe a method for detecting the presence or absence of at least one nucleotide sequence variation. This method is known as ARMS (amplification refractory mutation system). The basis of the invention as described in European Patent No. 332435 is that oligonucleotides with a mismatched 3'-residue will not function as primers in a PCR reaction under appropriate conditions. The method comprises (i) contacting a nucleic acid sample with a diagnostic primer which is substantially complementary to a diagnostic portion of a target nucleic acid sequence, whereby extension of the diagnostic primer on a target template under appropriate conditions is only achieved where a terminal nucleotide of the diagnostic primer is complementary to either a suspected variant nucleotide or a corresponding normal nucleotide of the target nucleic acid sequence, and (ii) detecting the presence or absence of an extension product. According to the invention described in European Patent No. 332435, a preferred method of detecting and distinguishing between multiple amplification products generated by the method comprises selecting the position of the amplification primers on the target nucleic acid sequence such that the length of each amplification product is different. Accordingly, this may be accomplished by varying the distance of the amplification primers from the position of the labeling primers such that each variant nucleotide is associated with an amplification product of different length. The amplification products of different length may then be detected by established electrophoretic techniques. The above described detection technique, however, has the disadvantage of being time-consuming and non-homogeneous, entailing several manual worksteps. Additionally, the technique may only be used for simultaneous screening of a sample of target nucleic acid sequences for either the presence or absence of variant nucleotides in multiple but different positions on said target nucleic acid sequences, whereas a simultaneous analysis of multiple variant nucleotides situated in a defined single position is not feasible. Furthermore, the invention described in European Patent No. 332435 does not allow for the quantification of target nucleic acid sequences.

Several improved PCR strategies for simultaneously identifying both alleles of a biallelic system have been described (Boldt et al., Brit J Haemat 1997, 99:968-973; Hamajima et al., Jpn J Cancer Res 2000, 91:865-868). For example, Boldt et al. (Brit J Haemat 1997, 99:968-973) describe a method based on ARMS, employing four primers and a two-annealing temperature cycling profile to simultaneously genotype both alleles of HPA-1 in a single PCR reaction. This method is capable of simultaneous amplification of both HPA-1 alleles (if present), but several drawbacks remain. First, the allele-specific primers have to be positioned at opposite template strands to enable generation and monitoring of two allele-specific PCR products. In addition, the polymorphism has to be off-center relative to the upstream and downstream external primers such that said generated allele-specific PCR products are of significantly different length in order to be accessible for the analysis by agarose gel electrophoresis, which is still required for monitoring of the PCR products. Thus, the method of Boldt et al. has the disadvantage of being time-consuming and non-homogeneous, entailing several manual worksteps. Additionally, the technique may only be used for simultaneous screening of a sample of target nucleic acid sequences for either the presence or absence of variant nucleotides in multiple but different positions on said target nucleic acid sequences, whereas a simultaneous analysis of multiple variant nucleotides situated in a defined single position is not feasible. Furthermore, the method does not allow for the quantification of target nucleic acid sequences. Further disadvantages of the method of Boldt et al. are the requirements for large amounts of genomic DNA (250 ng per reaction) and a two-step temperature profile.

The present invention features a homogeneous, sensitive, simple, and labor-saving technology for the multiplex analysis of nucleic acid sequences. The novel method is particularly useful for the direct detection of known SNPs, point-mutations, variant alleles, deletions, insertions, repeats and/or inversions. Additionally, the method of the present invention enables assaying the cytosine methylation status in chemically pretreated genomic DNA. The current invention features a method which utilizes the high specificities of semi-nested polymerase chain reaction (Haff, PCR Methods Appl 1994, 3:332-337) with an improved method of variant nucleotide-specific amplification. The improved variant nucleotide-specific amplification method is characterized by (1) the use of at least one, more preferably the use of at least two substantially similar but distinguishable diagnostic primers for the analysis of a target nucleic acid sequence, (2) by the combination of allele-specific hybridization and simultaneous extension of said diagnostic primers in a single reaction and (3) by the discrimination of non-extended from extended diagnostic primers. The inventive method offers the advantage over other systems in that it enables amplification, labeling, and monitoring in a homogeneous format. According to the present invention, multiple reaction products can be monitored, analyzed and quantified directly and simultaneously in a mix-and-measure format, without any post-amplification processing or physical separation steps. The technique of fluorescence correlation spectroscopy (FCS) is particularly well suited for such monitoring

The present invention provides a method for detecting the presence or absence of variant nucleotides within target nucleic acid sequences, said method comprising the following steps:

(i) setting up a reaction mix comprising nucleoside triphosphates or functional derivatives thereof, a polymerizing agent, at least one pair of target-specific amplification primers capable of hybridizing to target nucleic acid sequences, at least one set of diagnostic primers, each set consisting of at least two types of diagnostic primers capable of variable allele-specific hybridizing to corresponding target nucleic acid sequences 3'-relative to said amplification primers, such that each set of diagnostic primers is semi-nested relative to the corresponding pair of amplification primers, the at least two types of diagnostic primers of a set of diagnostic primers being characterized by having substantially similar nucleotide sequences except for at least one internally located nucleotide, which is different for each type of diagnostic primer and complementary to the variant nucleotide, whereby an extended diagnostic primer is being synthesized if said internally located nucleotide of the diagnostic primer is complementary to the corresponding nucleotide in the target nucleic acid sequence, and whereby substantially no or a negligible background of extended diagnostic primer is being synthesized when said internally located nucleotide is not complementary to the corresponding nucleotide in the target nucleic acid sequence, and each type of diagnostic primer further being characterized by carrying a different and distinguishable tag. The reaction mix further comprises at least one sample of target nucleic acid sequences.

(ii) performing an amplification reaction under conditions permitting hybridization of the amplification primers and variable allele-specific hybridization of the diagnostic primers, either together or sequentially, to the corresponding target nucleic acid sequences, and promoting polymerization; and

(iii) monitoring specific properties of said types of diagnostic primers, directly or indirectly, during or after completion of the amplification reaction, said specific properties being indicative for an extension or non-extension of the diagnostic primers, thereby detecting the presence or absence of a variant nucleotide contained within a target nucleic acid sequence.

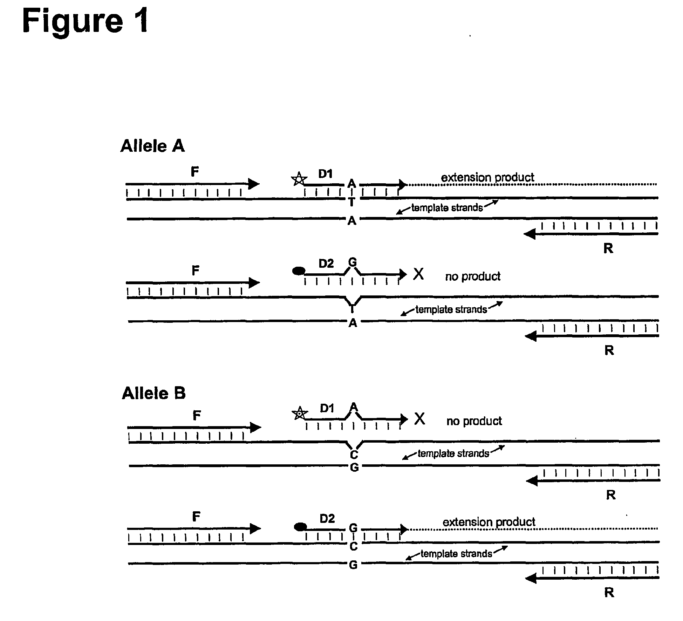

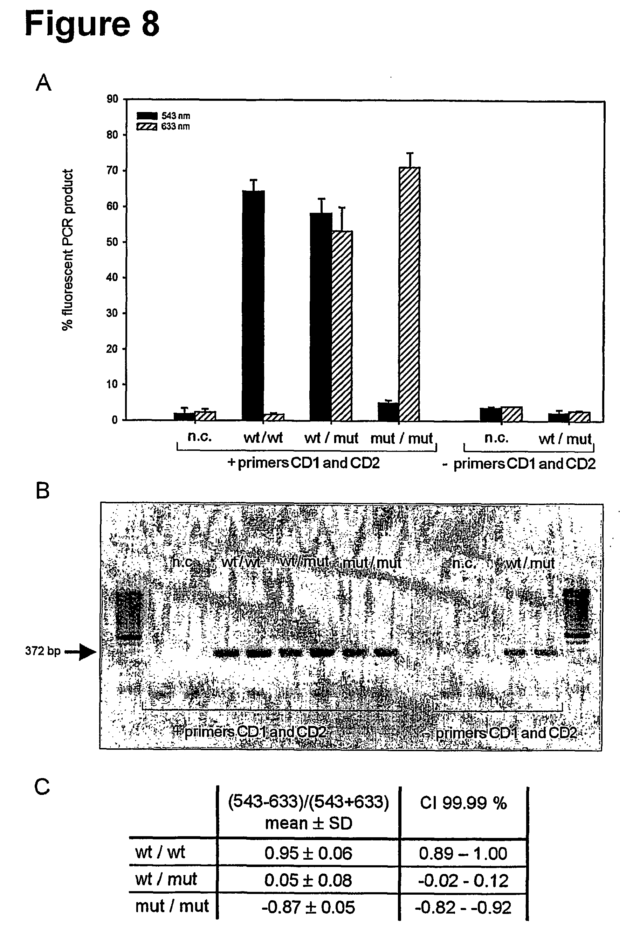

According to the featured method, it should be appreciated that an extension product of the diagnostic primer is synthesized when the internally located nucleotide of the diagnostic primer is complementary to the corresponding nucleotide of the target nucleic acid sequence. It should be further noted that the term "semi-nested", when referring to the diagnostic primer, means that each type of diagnostic primer anneals or hybridizes within the region of the target nucleic acid sequence that is framed and amplified by the pair of amplification primers. Each pair of said amplification primers consists of an upstream and corresponding downstream amplification primer, framing the region to be analyzed on the target nucleic acid sequence (see Figure 1 for illustration). The term "target nucleic acid sequence" as used herein is referring to a particular nucleic acid sequence of interest which is suspected of containing, or known to contain a variant nucleotide. The term "variant nucleotide" as used herein is defined as being a nucleotide contained within a target nucleic acid sequence at a defined position which is suspected of being, or known to be, variant. This means that a variant nucleotide may comprise either of four possible nucleotides (adenosine, guanosine, cytidine, and thymidine) or a modified nucleotide. In the practice of the invention, for example, a variant nucleotide may comprise a point mutation, deletion, insertion, inversion, a single nucleotide polymorphism (SNP) or a methylated nucleotide. Different alleles of a gene may be defined by the presence or absence of a variant nucleotide. It should further be appreciated that a sample of target nucleic acid sequences may contain a mixed population of target nucleic acid molecules, representing at least two different nucleic acid sequences, said target nucleic acid sequences differing at least in their nucleotide composition at the position defined by the corresponding complementary internally located nucleotide of the diagnostic primer. Consequently, the reaction mix may contain a mixture of at least two types of extended and amplified diagnostic primers, said multiple amplified types of diagnostic primers being distinguishable by virtue of their different tags. In practice, polymorphisms wherein more than two of the four possible nucleotides may be present at a given single-base location, are amenable to qualitative and quantitative analysis by the described method. The reaction mix also comprises nucleoside triphosphates or functional derivatives thereof. As used herein, nucleoside triphosphates or functional derivatives thereof are capable of being incorporated into an extension or amplification product. Functional derivatives of nucleoside triphosphates comprise, for instance, synthetic nucleotides having modified base moieties and/or modified sugar moieties. Principally, the method according to the invention can also be carried out by setting up a reaction mix comprising only one type of diagnostic primer (see Figure 8).

In a preferred embodiment, the present invention provides a method as hereinbefore described, wherein the concentration of each diagnostic primer is 0.5 to 0.001 times, preferably 0.25 to 0.01 times, most preferably 0.1 to 0.03 times the concentration of each amplification primer. The herein described method preferably employs at least one pair of target nucleic acid sequence-specific amplification primers at high concentrations (e.g. 75 to 500 nM) and at least two types of semi-nested, variant nucleotide-specific diagnostic primers at significantly lower concentrations (e.g. 1 to 50 nM) in a single amplification reaction, e.g. PCR. During the reaction, the target region containing the variable nucleotide of interest is amplified independently of the variant nucleotide sequence by the use of the amplification primers, whereas the nested diagnostic primers are hybridized and extended depending on the variant nucleic acid sequence. If the nucleic acid sequence of the diagnostic primer is complementary to the respective variant nucleic acid sequence to be analyzed, there will be a specific hybridization and subsequent efficient extension of said diagnostic primer by the polymerizing agent. To the contrary, if the internally located variant nucleotide of a diagnostic primer is not complementary to the respective nucleotide in the target nucleic acid sequence, proper hybridization and subsequent specific extension is impaired.

Several prior art methods which employ allele-specific hybridization principles to detect single nucleotide polymorphisms envisage significant unspecific, i.e., false-positive hybridization. The main reason for this unspecificity is that the discriminating principle of a hybridization probe, as commonly used, depends solely on hybridization thermodynamics. A single base pair mismatch within a region of 20 or more perfectly matched base pairs has significant influence on the hybridization kinetics only under restricted experimental conditions, i.e., temperature, salt and probe concentration. In general, low temperature, high salt concentration and high probe concentration increase hybridization efficiency but at the same time reduce the discrimination potential and favour false hybridization. Consequently, such a mismatched but still hybridized probe leads to false-positive hybridization signals.

The inventive method described here solves these drawbacks in the following way: The discriminating principle of the diagnostic primers of the present invention depends not solely on hybridization thermodynamics, but also on the differential extension efficiency of perfectly matched or mismatched primers by a polymerizing agent. This leads to the situation that mismatched and thereby weakly annealed diagnostic primers give no false-positive signal because they are not extended efficiently. In addition, the inventive method described here uses said variant nucleotide specific diagnostic primers at very low concentrations (down to 1 nM), which strongly favor discriminative hybridization and subsequent allele-specific extension.

Nevertheless, in the case of high DNA concentrations in the amplification reaction an unspecific amplification may occur. In order to further minimize such unspecific extension,

(1) the semi-nested diagnostic primers are present in significantly lower concentrations than the pair of amplification primers,

(2) the nucleotide sequences of the amplification primers and the semi-nested diagnostic primers can be selected in such a way that the amplification primers predictively hybridize to the template nucleic acid at significantly higher annealing temperatures than the semi-nested diagnostic primers,

(3) the thermal cycling profile for amplification can involve two segments of thermal cycles, the first segment of thermal cycles comprising a so-called touch-down step, said touch-down step being defined as a series of denaturation/annealing/elongation steps wherein the annealing temperature in each step is between 0.1°C and 2.5°C, more preferably between 0.2°C and 1°C lower than in the preceeding step, and the second segment of thermal cycles comprising a series of denaturation, annealing, and elongation steps wherein the annealing temperature in each step is kept constant at a temperature which may be identical to or below the annealing temperature of the last annealing step of the first segment of thermal cycles.

Optionally, the first segment of thermal cycles comprising the touch-down step can be refined by a combined touch-down/ time-up step. Said combined touch-down/ time-up step is defmed as a series of denaturation, annealing, and elongation steps wherein (a) the annealing temperature in each step is between 0.1°C and 2.5°C, more preferably between 0.2°C and 1°C lower than in the preceeding step and (b) at the same time, the annealing time in each step is between 0.1 sec and 5 sec, more preferably between 0.5 sec and 2 sec longer than in the preceeding step.

Due to their high concentration, the amplification primers dominate the first segment of thermal cycles, whereas the diagnostic primers, due to their low concentration and, optionally, to their lower annealing temperature, are extended predominantly during the second segment of thermal cycles in later PCR stages, thereby constituting a temporally and spatially semi-nested primer system. This feature has the advantage of leading to high signal-to-noise ratios, very high specificity and elimination of false-positive results.

In another preferred embodiment of the present invention, each diagnostic primer contains at least one of the following tags: fluorescent dyes, chemiluminescent tags, electroluminescent tags, magnetic tags, affinity or binding tags, nucleotide sequence tags, position specific tags, or tags with specific physical properties such as different size, mass, gyration, ionic strength, dielectric properties, polarization, or impedance.

In a further preferred embodiment of the present invention, the diagnostic primers contain fluorescent dyes, and each type of diagnostic primer is tagged with a different fluorescent dye, such that each type of diagnostic primer is characterized by different excitation- and/or emission spectra, life-time properties, polarization properties, fluorescence resonance enery transfer (FRET) properties, quantum yields, photostability, or triplet number of fluorochromes, or different electro-osmotic or electrophoretic properties. It may be preferred to use a variety of different labels in combination, such that they encode and represent multiplex complexity (e.g. dye combinations as used in bar-coding of beads).

In another preferred embodiment of the method of the present invention, said tag is permanently or temporarily attached to either the 3'-end or the 5'-end of the diagnostic primer, preferably to the 5'-end of the diagnostic primer. The methods of linking, attaching, or conjugating the tag to the diagnostic primer depend on the type of tag and the position of the tag on the diagnostic primer and are commonly known in the art. Diagnostic primers and amplification primers are comprised of oligonucleotides which can be of any suitable size, preferably in the range of 5-100 or 5-50 nucleotides, more preferably in the range of 5-40 nucleotides. However, depending on the particular complexity of the target sequence, the annealing temperature, and other variable factors, the primers may contain more or fewer nucleotides.

In a further preferred embodiment of the present invention, a diagnostic primer contains a nucleotide sequence tag, and each type of diagnostic primer is tagged with a different and distinguishable nucleotide sequence tag. Such a nucleotide sequence tag shall be referred to hereinafter as a "5'-tail". The nucleotide sequence of such a 5'-tail shall be referred to as "5'-tail sequence". The nucleotide sequence tag of a diagnostic primer shall be referred to as "5'-tail sequence portion", or simply "5'-tail portion". Preferably, said nucleotide sequence tag is permanently attached to the 5'-end of a diagnostic primer, such that said diagnostic primer becomes a "chimeric diagnostic primer". Chimeric diagnostic primers consist of a 3'-nucleotide sequence portion, hereinafter referred to as "diagnostic primer portion", which is complementary to the target nucleotide sequence in a variant nucleotide specific manner and of a 5'-tail sequence portion, which is preferably non-complementary to the target nucleic acid sequence and functions as a detectable and distinguishable 5'-tail in this embodiment. The 5'-tail sequence portion of said chimeric diagnostic primer may be of any suitable length, preferably in the range of 5-50 nucleotides, more preferably in the range of 10-25 nucleotides. Upon variant nucleotide specific extension of the chimeric diagnostic primers and further cycles of amplification, the nucleotide sequences comprising the 5'-tail portion of said chimeric diagnostic primers as well as the respective complementary sequences of said 5'-tail portions become an integral part of the resulting amplified extension products.

In a further preferred embodiment using said chimeric diagnostic primers, the presence or absence of each type of the different, detectable, and distinguishable 5'-tail sequence portion, i.e. the 5'-tail, of said chimeric diagnostic primers in or as part of the amplified extension products is detected by including in the reaction a corresponding type of 5'-tail specific probe. Said 5'-tail specific probe can be a "5'-tail specific labeling primer" or a "5'-tail specific hybridization probe" or both.
A 5'-tail specific labeling primer hybridizes to a complementary 5'-tail sequence portion of a chimeric diagnostic primer and is extended in subsequent elongation or amplification reactions, and thus functions as a primer for nucleotide polymerization by a polymerizing agent, whereas a 5'-tail specific hybridization probe hybridizes to a complementary 5'-tail sequence portion of a chimeric diagnostic primer but is not extended by a polymerizing agent and does not function as a primer.

Further, said 5'-tail specific probe contains at least one of the following tags, i.e. labels: fluorescent dyes, chemiluminescent tags, electroluminescent tags, magnetic tags, affinity or binding tags, position specific tags, or tags with specific physical properties such as different size, mass, gyration, ionic strength, dielectric properties, polarization, or impedance, whereby said tag is permanently or temporarily attached to the respective 5'-tail specific probe. Preferably, the tag is attached to the 5'-end of said 5'-tail specific probe.

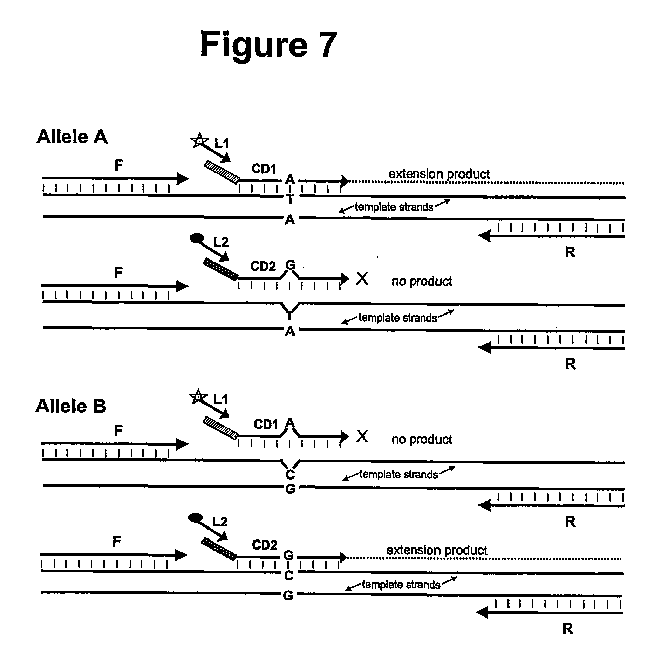

In another preferred embodiment, using said chimeric diagnostic primers in conjunction with a 5'-tail specific probe, said 5'-tail specific probe contains fluorescent dyes, and each type of 5'-tail specific probe is tagged with a different fluorescent dye, such that each type of 5'-tail specific probe is characterized by different excitation-and/or emission spectra, life-time properties, polarization properties, fluorescence resonance enery transfer (FRET) properties, quantum yields, photostability, or triplet number of fluorochromes, or different electro-osmotic or electrophoretic properties. It may be preferred to use a variety of different labels in combination, such that they encode and represent multiplex complexity (e.g. dye combinations as used in bar-coding of beads). This embodiment, specifically for the use of 5'-tail specific labeling primers as 5'-tail specific probes (designated as L1 and L2), is schematically illustrated in Figure 7.
Chimeric diagnostic primers, 5'-tail specific probes, for instance 5'-tail specific labeling primers or 5'-tail specific hybridization probes, and amplification primers are comprises of oligonucleotides which can be of any suitable size, preferably in the range of 5-100 or 5-50 nucleotides, more preferably in the range of 5-40 nucleotides. However, depending on the particular complexity of the target sequence, the annealing temperature, and other variable factors, the primers or probes may contain more or fewer nucleotides.

In a more preferred embodiment using said chimeric diagnostic primers in conjunction with a 5'-tail specific hybridization probes as a 5'-tail specific probe, the reactions are performed in the presence of surface-immobilized oligonucleotide sequences which constitute complementary, or substantially complementary, capture probes for said 5'-tail specific hybridization probes. By executing the amplification reaction in the presence of said chimeric diagnostic primers, 5'-tail specific hybridization probes and surface-immobilized capture-probes, the 5'-tail sequence portion of the chimeric diagnostic primer as part of the amplified extension products and said surface-immobilized capture-probes represent competitive hybridization targets for said 5'-tail specific hybridization probes. Hence, the presence or absence of each type of said 5'-tail sequence portions of the amplified extension products can be detected by monitoring the varying amount of fluorescent hybridization probe annealed to the solid surface. Preferably, said 5'-tail specific hybridization probe is a labeled hybridization probe, most preferably a fluorescent hybridization probe. Preferably, said solid surface is for example the wall of a reaction tube or vessel, a slide surface or a bead surface, whereby a reaction tube or vessel, or slide surface may be composed of the group of materials including but not limited to glass, polymer or silica, or mixtures thereof, whereby a bead surface may be comprised by microparticles and/or nanoparticles, and/or mixtures thereof, whereby micro- and/or nanoparticles are composed from the group of materials including but not limited to glass, silica, polymers, magnetic or paramagnetic materials, or mixtures thereof, whereby the diameter of micro- and/or nanoparticles is between 0.01 and 10 $\mu$m, more preferably between 0.05 and 5 $\mu$m, even more preferably between 0.3 and 3 $\mu$m.

In a preferred embodiment of the present invention, the diagnostic primers, chimeric diagnostic primers, 5'-tail specific probes, for instance 5'-tail specific labeling primers or 5'-tail specific hybridization probes, and amplification primers comprise phosphodiester oligonucleotides or modified oligonucleotides such as methylphosphonates, phosphotriesters, phosphorothioates, phosphoramidates, PNAs, LNAs, non-phosphate internucleoside linkages or mixtures thereof.

The use in the embodiments of the present invention of chimeric diagnostic primers in conjunction with 5'-tail specific probes has the advantage that a given 5'-tail sequence portion of a chimeric diagnostic primer can be combined with any target-specific diagnostic portion to create a multitude of chimeric diagnostic primers which all contain said given 5'-tail sequence portion. Such a given 5'-tail sequence portion can be universally used in applications that employ a multitude of different chimeric diagnostic primers. Consequently, a multitude of amplified extension products from said multitude of chimeric diagnostic primers contain a universal 5'-tail sequence portion, the presence or absence of which can be monitored by adding a single type of universal 5'-tail specific probe, for instance a 5'-tail specific labeling primer or a universal 5'-tail specific hybridization probe. Likewise, by using two types of different and distinguishable universal 5'-tail sequence portions, two types of multitudes of amplified extension products carrying two types of different and distinguishable universal 5'-tail sequence portions can then be monitored by adding two types of different and distinguishable universal 5'-tail specific probes, for instance a 5'-tail specific labeling primer and/or a universal 5'-tail specific hybridization probe.

It is easily appreciated that the use of said two types of universal 5'-tails and their corresponding universal 5'-tail specific probes, according to the present invention, enables the fast and economical development of assays for the high-troughput analysis of bi-allelic SNPs because the need for the synthesis of labeled primers and/or probes for each individual SNP target, as is the case for many prior art techniques, e.g. TaqMan assays or LightCycler assays, is no longer necessary. The synthesis of fluorescently labeled primers and/or probes is expensive and time-consuming and hence represents a major limitation during assay development of a large number of (up to hundred-thousands) SNP assays as required for high-throughput SNP genotyping. Synthesis of fluorescently labeled primers and/or probes is a major cost driver for SNP genotyping, particularly because in most high throughput SNP genotyping programs a plurality of SNP targets (up to many thousands) are analyzed for a relatively small number of genomic DNA samples. Besides the high cost and time requirements for fluorescent labeling, fluorescent dyes that are permanently attached to oligonucleotide primers or probes influence the hybridization properties of said oligonucleotide primers or probes. Said potential adverse effects on hybridization properties are difficult to predict. Using fluorescently labeled universal 5'-tail specific labeling primer and/or 5'-tail specific hybridization probes, said adverse effects have to be calibrated only once. Likewise, SNPs consisting of more than two alleles or multiple bi-allelic SNPs can be addressed by using three or more types of different and distinguishable universal 5'-tail sequence portions in combination with the corresponding three or more types of different and distinguishable universal 5'-tail specific probes.

In yet another preferred embodiment of the present invention, the amplification reaction and subsequent simultaneous monitoring of the specific properties of the multiple reaction products is performed in a homogeneous format, i.e. in a single reaction tube. It is easily appreciated that this embodiment has the advantage of increased sample throughput and potential cost and time savings.

In another embodiment of the present invention, the amplification reaction and subsequent simultaneous monitoring of the specific properties of the multiple reaction products is performed in a non-homogeneous format, said non-homogeneous format being characterized by performing said reactions on a solid surface, said solid surface being for example the wall of a reaction tube or vessel, a slide surface or a bead surface, whereby a reaction tube or vessel or slide surface may be composed of the group of materials including but not being limited to glass, polymer or silica, or mixtures thereof, whereby a bead surface may be comprised by microparticles and/or nanoparticles, and/or mixtures thereof, whereby micro- and/or nanoparticles are composed of the group of materials including but not being limited to glass, silica, polymers, magnetic or paramagnetic materials, or mixtures thereof, whereby the diameter of micro- and/or nanoparticles is between 0.01 and 10 $\mu$m, more preferably between 0.05 and 5 $\mu$m, even more preferably between 0.3 and 3 $\mu$m.

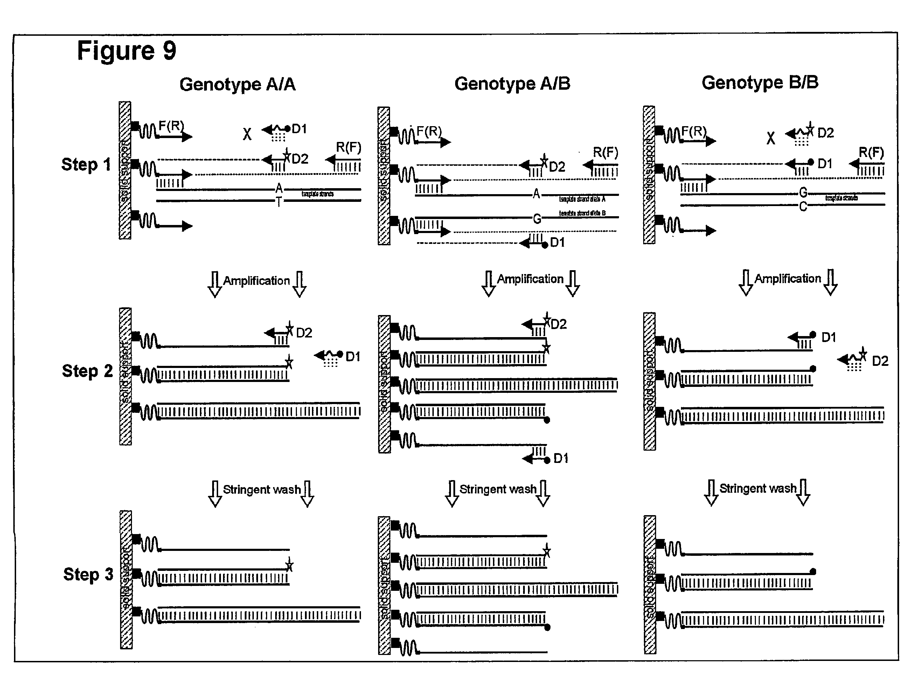

In a preferred embodiment of the non-homogeneous format of the invention, at least one primer of the at least one pair of amplification primers is immobilized to a solid surface, either permanently or temporarily, preferably at its 5'-terminus (Figure 9). The immobilization of said at least one primer of the at least one pair of amplification primers to said solid surface may be performed by techniques commonly known in the art, such as carbodiimide coupling, avidin-biotin coupling, or other means. In another preferred embodiment said immobilization of said at least one amplification primer on said solid support is preferably performed in a microarray format, said microarray format being defined as a one-dimensional, more preferably a two-dimensional positional arrangement of spots containing said immobilized primer(s). Oligonucleotide microarrays may be fabricated by applying said primers to microchip surfaces by different methods described in the art such as pin transfer (Guschin et al., Anal. Biochem. 1997, 250:203-211), inkjet printing (Blanchard et al., Biosens. Bioelectron. 1996, 11:687-690) or by parallel oligonucleotide synthesis directly on a support (Fodor et al., Science 1991, 251:767-773; McGall et al., Proc. Natl. Acad. Sci. USA 1996, 93:13555-13560) or by copolymerization of oligonucleotides in microarrays of polyacrylamide gel pads (Vasiliskov et al., BioTechniques 1999, 27:592-606). It is easily appreciated that this embodiment allows the parallel analysis of many different nucleic acid variants and has the advantage of potential cost and time savings. In another preferred embodiment, said immobilization of said at least one amplification primer on said solid support is preferably performed in a liquid microarray format, said liquid microarray format being defined as a set of said micro-and/or nanoparticles, said set of micro- and/or nanoparticles being composed of at least two, more preferably 5-20, and even more preferably 10-100 homogeneous subsets of micro- and/or nanoparticles, each individual homogeneous subset of micro-and/or nanoparticles carrying an individual type of said immobilized at least one amplification primer(s), and said individual homogeneous subsets of micro- and/or nanoparticles being distinguishable from each other by physical properties such as size, shape, specific density, light scattering, colour, fluorescence properties or other characteristics. It is easily appreciated that this embodiment allows the parallel analysis of many different nucleic acid variants and has the advantage of potential cost and time savings.

In a further embodiment of the present method the polymerizing agent is an enzyme. The synthesis of primer extension products can be accomplished, for instance, by E.coli DNA polymerase I, Klenow polymerase, phage T4 DNA polymerase, or other DNA polymerases. A preferred enzyme is a thermostable DNA polymerase. It is particularly desirable to use a thermostable DNA polymerase which lacks 5'- to 3'-exonuclease activity, e.g., a truncated form of *T. aquaticus* DNA polymerase (Lawyer et al., J Biol Chem 1989, 264:6427-6437; Lawyer et al., PCR Method Appl 1993, 2:275-287). By using such an enzyme the diagnostic primers are not subject to exonuclease degradation from the tag-carrying 5'-terminal end of the diagnostic primer during the amplification reaction.

In another embodiment of the present invention the target nucleic acid sequences comprise preferably genomic DNA. Additionally, target nucleic acid sequences may comprise cDNA, single stranded DNA, double stranded DNA, plasmid DNA, mixtures of DNA with other molecules. Furthermore, target nucleic acid sequences may be comprised of RNA. When RNA is the starting material for the analysis, a reverse transcription reaction and an amplification reaction may be performed in the same reaction. The origin of the target nucleic acid sequences is preferably human, but it may also originate from other organisms, such as other mammals, vertebrates, invertebrates, fungi, yeast, bacteria, viruses, and plants. The target nucleic acid sequences may be obtained and extracted preferably from blood. It may further be obtained from other accessible tissue material and body fluids such as tissue biopsies, tumor material, skin, hair, sperm, saliva, cord blood, cerebrospinal fluid, and amniotic fluid. Yet another source of target nucleic acid sequences may be from cell culture material. Extraction may be performed by a number of techniques known to those of ordinary skill in the art (see Sambrook and Russell, Molecular Cloning: A Laboratory Manual, Cold Spring Harbor Laboratory Press, Cold Spring Harbor, New York, 2000).

In a further preferred embodiment of the invention the monitoring of the specific properties of the amplification reaction products may be determined by a number of different techniques. For instance, chromatographic techniques such as HPLC, FPLC, capillary elecrophoresis, or gel electrophoresis may be employed. Mass spectroscopic or electrochemical techniques may also be used. The monitoring can be performed during (i.e., real time analysis) or after the completion of the PCR-amplification (i.e., end point analysis). Depending on the nature of the tag attached to the diagnostic primers, and/or the 5'-tail specific probe the invention allows the use of fluorimetric, chromatographic, or physical detection systems to measure the degree of extension of the diagnostic or chimeric diagnostic primers. In the preferred embodiment, fluorimetric analysis is desired for the monitoring of the amplification reaction products. The monitoring can be based on fluorescence polarization analysis, fluorescence anisotropy analysis, fluorescence intensity analysis, fluorescence intensity distribution analysis, fluorescence lifetime analysis, fluorescence dichroism analysis, fluorescence resonance energy transfer analysis, spectroscopic analysis of excitation and/or emission spectra, or, preferably, fluorescence correlation spectroscopy (FCS). It may also be based on combinations of these fluorescence based techniques. Furthermore, fluorimetric analysis may be carried out in a confocal fluorescence system. For multiplex analysis of a homogeneous amplification reaction, fluorescent tags with distinguishable optical parameters such as excitation spectra, emission spectra, fluorescence life-time, polarization, quantum yields, photostability, or triplett number of fluorochromes are used. In the case of size or mass tags, spectroscopy is a preferred detection system. It may also be preferred to use said tags in combination.

In one embodiment of the invention, monitoring the degree of extension of a diagnostic or chimeric diagnostic primer comprises the qualitative detection of variant nucleotides contained within a sample of target nucleic acid sequences. According to this embodiment, an extended diagnostic or chimeric diagnostic primer is indicative for the presence of a variant nucleotide, and a non-extended diagnostic or chimeric diagnostic primer is indicative for the absence of a variant nucleotide.

In another embodiment of the invention, monitoring the amounts of extended diagnostic or chimeric diagnostic primers comprises the quantification of target nucleic acid sequences. This feature is particularly useful for genotyping samples of target nucleic acid sequences. According to this embodiment quantification may, for instance, be achieved by (i) calculating the ratio of the values of individually recorded signals (signal A/ signal B), or (ii) by subtracting the value of signal B from the value of signal A (signal A - signal B), or (iii) by dividing the value of the remainder of signal A after subtraction of signal B by the value of the sum of signal A and signal B ((signal A - signal B) / (signal A + signal B)).

According to one embodiment of the present invention, the method may be used for detecting the presence or absence of one or more variant nucleotides associated with an inherited or acquired condition or disease.

Additionally, according to another embodiment of the present invention, the method may be employed for the diagnostic screening of many samples of target nucleic acid sequences for inherited or acquired conditions or diseases. The method further allows for the screening for the presence or absence of one or more variant nucleotides that may be indicative for a predispositon of acquiring a certain condition or disease.

In another embodiment, the method of the present invention enables the analysis of the cytosine methylation status in chemically pretreated genomic DNA. Methylation of a cytosine located 5' to a guanosine is known to effect the expression of several eukaryotic genes (Bird, Cell 1992, 70: 5-8). In normal cells, methylation occurs predominantly in CG-poor regions, while CG-rich areas, called "CpG islands," remain unmethylated. The exception is extensive methylation of CpG islands associated with epigenetic phenomena such as the transcriptional inactivation of regulatory regions of imprinted genes, i.e. genomic imprinting (Li et al., Nature 1993, 366: 362-365) and with the complete inactivation of entire genes on the X-chromosome of females (Pfeifer et al., Science 1989, 246: 810-813). Aberrant methylation of normally unmethylated CpG islands has been described as a relatively frequent event in immortalized and transformed cells (Antequera et al., Cell 1990, 62: 503-514) and has been associated with transcriptional inactivation of defined tumor suppressor genes in human cancers (Herman et al., Proc Natl Acad Sci USA 1996, 93: 9821-9826). Sensitive detection of CpG island methylation has the potential to define tumor suppressor gene function and provides a new strategy for early tumor detection. Besides the occurrence in many common human cancers, epigenetic changes have been found in a series of other common diseases. Therefore, an altered pattern of methylation may reflect the development of certain human diseases, or may indicate the predisposition to develop such diseases. Thus a specific epigenetic fingerprint may be an early warning diagnosis of many human diseases and disorders.

Methylation-specific PCR (Herman et al., Proc Natl Acad Sci USA 1996, 93: 9821-9826) entails initial modification of DNA by sodium bisulfite which converts unmethylated cytosine to uracil, whereas methylated cytosine remains unaffected (Wang et al., Nucleic Acids Res 1980, 8:4777-4790). Consequently, a methylated DNA sequence will differ from an identical but unmethylated DNA sequence after bisulfite treatment. Patent application WO 02/04686 describes one example of use of the modification of DNA with a bisulfite salt to analyze the methylation status of cytosine bases.

Hence, the method of the present invention enables assaying the methylation status of DNA, the determination of a specific epigenetic fingerprint of a given nucleic acid sequence, by designing different types of semi-nested diagnostic primers according to the present invention to specifically address each of those sequences; for example, a semi-nested diagnostic primer to amplify unmethylated, bisulfite treated DNA will have one or more G residues replaced by an A residue (to be complementary to uracil nucleotides that were previously unmethylated cytosine), and one or more C residues replaced by a T residue, respectively, relative to the corresponding semi-nested diagnostic primer to amplify methylated or untreated (wild type) DNA.

According to the method of the present invention, semi-nested diagnostic or Chimeric diagnostic primers are preferably specific for bisulfite-treated methylated, bisulfite-treated unmethylated, and nonbisulfite-treated (wild type) nucleic acids, respectively, and may be used either in separate reaction vessels or in variable combinations in a single reaction vessel.

Prior to the PCR amplification reaction, a sample containing purified nucleic acids is contacted with an amount of bisulfite sufficient to convert unmethylated cytosines to uracil. Upon conducting the amplification reaction in the presence of primer sets specific for preselected target sequences, the methylation status of the nucleic acids in the sample can be deduced from the monitoring of extended and amplified semi-nested diagnostic or chimeric diagnostic primers.

In a further embodiment of the present invention, the method may be used for quantifying target nucleic acid sequences, in particular for genotyping target nucleic acid sequences. The method may also be useful for the determination of the concentration of DNA or RNA, mRNA, or viral DNA or RNA in a sample. The use of the method for quantification purposes may be preferably desirable for the determination of the copy-number of target nucleic acid sequences, in particular in samples derived from tumor tissue. In the practice of the invention, in order to increase the quantification range of the method, a sample of target nucleic acid sequences may be mixed with a defined amount of engineered internal standard nucleic acids. All of the present nucleic acid sequences are then co-amplified by a polymerase-chain-reaction (PCR) or reverse transcription / polymerase-chain-reaction (RT-PCR) using identical pairs of amplification primers but target- and internal standard-specific diagnostic or chimeric diagnostic primers. The different types of diagnostic or chimeric diagnostic primers and/or labeling primers and/or hybridization probes within the reaction are distinguished by their tags. The amounts of both types of amplification products are determined by e.g. fluorescence or chromatographic read out When the amplification kinetic enters the plateau phase the enzymatic amplification efficiency of both targets is reduced. The comparison of the internal standard-specific signal with and without target nucleic acid sequences reflects the amplification efficiency of the total system. A correlation of the amount of amplified products and total amplification efficiency allows for an extensions of the quantification range.

In a second aspect, the present invention provides a use of a kit for detecting the presence or absence of variant nucleotides within a sample of target nucleic acid sequences in a method according to the invention, said kit comprising: nucleoside triphosphates or functional derivatives thereof, a polymerizing agent, at least one pair of target-specific amplification primers capable of hybridizing to a target nucleic acid sequence, at least one set of diagnostic primers, each set consisting of at least two types of diagnostic primers capable of variable allele-specific hybridizing to a corresponding target nucleic acid sequence internal to said amplification primers, such that each set of diagnostic primers is semi-nested relative to the corresponding pair of amplification primers, the at least two types of diagnostic primers of a set of diagnostic primers being characterized by having substantially similar nucleotide sequences except for at least one internally located nucleotide, which is different for each type of diagnostic primer and complementary to the variant nucleotide, whereby an extended diagnostic primer is being synthesized when said internally located nucleotide of the diagnostic primer is complementary to the corresponding nucleotide in the target nucleic acid sequence, and each type of diagnostic primer further being characterized by carrying a different and distinguishable tag.

In a preferred embodiment, the diagnostic primers of said kit are chimeric diagnostic primers. Optionally, said kit may comprise 5'-tail specific probes. Furthermore, said kit may comprise universal 5'-tail-specific labeling primers and/or universal 5'-tail specific hybridization probes. It may be desirable to include internal standard nucleic acid sequences in the kit. Such internal standards are engineered nucleic acid sequences being substantially identical to an amplified target nucleic acid sequence. In order to ensure comparable enzymatic amplification properties of target nucleic acid sequence and corresponding internal standard nucleic acid sequence, the altered sequence region should be as small as possible, e.g. 1 to 50 nucleotides, preferably 1 to 20, most preferably 1 to 4 nucleotides. In case of longer stretches of altered sequence, the sequence should be scrambled. This means that the nucleic acid sequence is altered in such a way that the overall nucleotide composition (G,C,A,T-content) remains substantially unchanged. In case of DNA target nucleic acid sequences, a DNA internal standard is preferred, in case of RNA target nucleic acid sequences, an RNA internal standard is preferred.

The kit of the present invention can be used for detecting the presence or absence of one or more variant nucleotides associated with an inherited or acquired condition or disease, for genotyping, for quantification of target nucleic acid sequences, and for assaying the methylation status, preferably the status of cytosine methylation of target nucleic acid sequences.

The above and other features and advantages of the invention will be apparent from the following description of figures and examples which are illustrative only and not intended to limit the remainder of the disclosure in any way.

The method of the present invention is illustrated by means of the example analysis of the SNP at position 655 ($A_{655}G$) within the human MLH1 (mutL homolog 1) gene (Papadopoulos et al., Science 1994, 263:1625-1629). The presence of the A to G transition in the genomic DNA causes the replacement of isoleucine at position 219 by valine (I219V) in the corresponding protein. The hMLH1 protein functions as a mismatch repair protein and helps to maintain the fidelity of DNA during replication. Germline mutations in mismatch repair genes such as the $A_{655}G$ transition within the hMLH1 gene account for the inherited familial cancer syndrome of hereditary non-polyposis colorectal cancer (HNPCC), in which affected individuals show accelerated development of cancers of the proximal colon, endometrium, and stomach (Liu et al., Nat Genet 1995, 9:48-55; Tannergard et al., Cancer Res 1995, 55:6092-6096; Liu et al., Nat Med 1996, 2:169-174).

Examples

The specificity, reproducibility, precision, and robustness of the inventive method are demonstrated by the analysis of the SNP at position 655 ($A_{655}G$) within the human MLH1 (mutL homolog 1) gene (Papadopoulos et al., Science 1994, 263:1625-1629).

Experimental Methods:

(1) Blood samples and DNA preparation: EDTA blood samples were received from a blood bank and stored at -20°C. Genomic DNA was recovered from whole blood using the QIAamp DNA Blood Mini Kit (QIAGEN, Hilden, Germany) according to the manufacturer's instructions. DNA was quantified by measuring the absorbance at 260 nm and purity of nucleic acids was monitored by controlling the 260 nm / 280 nm absorbance ratios.

(2) Primer design: All primers disclosed in the present invention were designed using Primer Premier Software, Version 5.00 (Premier Biosoft International, Palo Alto, CA, USA). Default settings in the program were used for reaction conditions, and the following parameters were set for primer selection: primer length = 20 +/- 5 bp; primer melting temperature (Tm) range = 47° - 62°C, primer GC-content = 40 - 65 %. Additional parameters were set when necessary to optimize primer selection, in particular for the selection of diagnostic or chimeric diagnostic primers. Allele-specific diagnostic or chimeric diagnostic primers were positioned at the polymorphic site. The sequences of the selected primers are as follows:

forward amplification primer (F): 5'-CTGATGTTAGGACACTACCCAATG-3';
reverse amplification primer (R): 5'-AGAAGGAGCATTGGAGGATAA-3';
wildtype-specific diagnostic primer (D1): 5'- GCTCC**A**TCTTT-3';
mutant-specific diagnostic primer (D2): 5'- GCTCC**G**TCTTT-3',
wildtype-specific chimeric diagnostic primer (CD1): *5'-AGGTCACTCGCACAGA*TG**TCGCTCCATCTTTGG**-3';
mutant-specific chimeric diagnostic primer (CD2): *5'-AGGTAACCACGCTCAATG***CGCTCCGTCTTTG-3'**;
5'-tail specific labeling primer for CD1 (L1): *5'-AGGTCACTCGCACAGATG*-3';
5'-tail specific labeling primer for CD2 (L2): *5'-AGGTAACCACGCTCAATG*-3'.

All primers were synthesized by ThermoHybaid (Interactiva Division,Ulm, Germany). Wildtype-specific diagnostic primer D1 and 5'-tail specific labeling primer L1 were 5'-tagged with TAMRA by standard phosphoramidite chemistry. Mutant-specific diagnostic primer D2 and 5'-tail specific labeling primer L2 were linked to N-hydroxysuccinimide-activated EVOblue-50 (EVOTEC OAI, Hamburg, Germany) via a C6-linker at the 5'-terminus.

(3) Assay conditions for genotyping by the method of invention: All PCR reactions were carried out in a final volume of 50 $\mu$l using the GeneAmp PCR System 9700 (PE Applied Biosystems, Weiterstadt, Germany). DNA samples were amplified in duplicates and analyzed by FCS directly after cycling without any purification steps. The reactions consisted of 1x amplification buffer [20 mM Tris-HCl, pH 8.9, 10 mM KCl, 10 mM $(NH_4)_2SO_4$, 100 $\mu$g/ml BSA, 0.05% Tween-20], 3 mM $MgCl_2$, 0.2 mM of each dNTP, 1 U Q-BioTaq (Q-BIOgene, Heidelberg, Germany) and 10 ng genomic DNA. Concentrations of the amplification primers were either 300 nM or 100 nM, concentrations of the diagnostic primers and chimeric diagnostic primers were 10 nM, and the concentration of the 5'-tail specific labeling primers were 5 nM. The following profile was used for thermal cycling of all reactions using labeled diagnostic primers D1 and D2: initial 3 min denaturation at 94°C, followed by 12 cycles consisting of denaturation at 94°C for 40 sec, annealing at 58°C (- 0.5°C / cycle) for 30 sec (+ 1 sec / cycle) and elongation at 72°C for 45 sec, followed by 35 cycles consisting of denaturation at 94°C for 40 sec, annealing at 46°C for 40 sec and elongation at 72°C for 45 sec. Samples were maintained at 72°C for 5 min before holding at 4°C prior to analysis.
The following profile was used for thermal cycling of all reactions using chimeric diagnostic primers CD1 and CD2 in conjunction with 5'-tail specific labeling primers L1 and L2: initial 3 min denaturation at 94°C, followed by 45 cycles consisting of denaturation at 94°C for 20 sec, annealing at 55°C for 40 sec and elongation at 72°C for 45 sec. Samples were maintained at 72°C for 5 min before holding at 4°C prior to analysis.

(4) Assay conditions for PCR allele-specific restriction analysis (PCR-ASRA): For hMLH1 genotyping, a PCR reaction was performed in a final volume of 50 $\mu$l consisting of: 1x amplification buffer [20 mM Tris-HCl, pH 8.9, 10 mM KCl, 10 mM (NH4)2SO4, 100 $\mu$g/ml BSA, 0.05% Tween-20], 2 mM $MgCl_2$, 0.2 mM of each dNTP, 300 nM sense primer (5'- GTTTGCTGGTGGAGATAAGG-3'), 300 nM antisense primer (5'-ATAGGTTATCGACATACCGACTAACAGCATTTCCATCGA-3'), 1 U Q-BioTaq and 100 ng genomic DNA of homozygous wildtype (wt/wt), heterozgous (wt/mut), or homozygous mutant (mut/mut) allelic state, respectively. PCR amplification was achieved using the following cycling conditions: initial denaturation for 2 min at 94°C followed by 40 cycles consisting of 94°C for 30 sec, 56°C for 30 sec, and 72°C for 40 sec. Finally, an elongation step at 72°C for 5 min was performed. The nucleotide sequence of the antisense primer was designed such that the amplified 191 bp PCR product contained a *ClaI* restriction site (AT/CGAT) when amplification was performed from a wt DNA, but did not contain a *ClaI* restriction site when amplification was performed from a mutant DNA.
15 $\mu$l of each amplification reaction were digested with 10 U *ClaI* I (New England Biolabs GmbH, Frankfurt, Germany) at 37°C for 2 hours in a final volume of 30 $\mu$l. 15 $\mu$l of digested samples and 5 $\mu$l of undigested PCR products were analyzed in parallel by electrophoresis in a 3 % TAE-agarose gel stained with ethidium bromide. A 100-bp DNA ladder (Life Technologies, Karlsruhe, Germany) was used as size marker.

(5) Data acquisition and analysis: After thermal cycling, 5 μl of each PCR reaction mix were analyzed by agarose-gel electrophoresis to monitor successful PCR amplification. 40 μl of each PCR reaction mix were transferred to 96-well microplates (UniView, Whatman via Merck Eurolab, Bochum, Germany) without any post-PCR-modification. FCS measurements were performed on a modified confocal microscope equipped with helium-neon lasers and filter sets for excitation at 543 nm and 633 nm). Each PCR reaction mix was measured 3 times, 3 seconds each, at both 543 nm and 633 nm. Alternatively, each PCR reaction mix was measured once for 3 seconds at both 543 nm and 633 nm. From the photon count signal, the autocorrelation curve was automatically calculated and evaluated with the autocorrelation function G($\tau$) using a two-component model corresponding to free and extended primer (Schwille et al., Biophys J 1997, 72:1878-1886):

$$G(\tau) = \left[1 - T + T \exp\left(\frac{-\tau}{\tau_T}\right)\right] N^{-1} \left[\frac{1-Y}{\left(1+\dfrac{\tau}{\tau_{free}}\right)\sqrt{1+\dfrac{r_0^2}{z_0^2}\dfrac{\tau}{\tau_{free}}}} + \frac{Y}{\left(1+\dfrac{\tau}{\tau_{poly}}\right)\sqrt{1+s^2\dfrac{\tau}{\tau_{poly}}}}\right]$$

where T is the average fraction of dye molecules in the triplet state with relaxation time $\tau$; N is the total average number of fluorescent molecules in the observation volume; Y is the relative fraction of extended primer; $\tau_{free}$ and $\tau_{poly}$ define the average diffusion times for free primers and extended primers (i.e., the PCR product), respectively, through the confocal observation volume. The parameter $S = r_0/z_0$ describes the ratio of the radius to the length of the detection area, where $r_0$ and $z_0$ are lateral and axial radii defining the football-like confocal observation volume (i.e., the distances between the coordinate where the Gaussian distribution of the emission light reaches its maximum value and the point where the light intensity decreases to $1/e^2$ of this maximum value). FCS data evaluation was performed automatically by analysis software and provided, among other fluorescence parameters, translational diffusion times and relative fractions of fluorescent primers and PCR products. Mean values for the relative fraction of PCR product were calculated from two or six measurements (resulting from duplicate PCR reactions, each measured once or three times, rerspectively). This was done separately for measurements obtained at 543 nm or 633 nm. Finally, the value ((signal A - signal B) / (signal A + signal B)) was calculated from the corresponding means of each (duplicate) probe.

Results and Analysis:

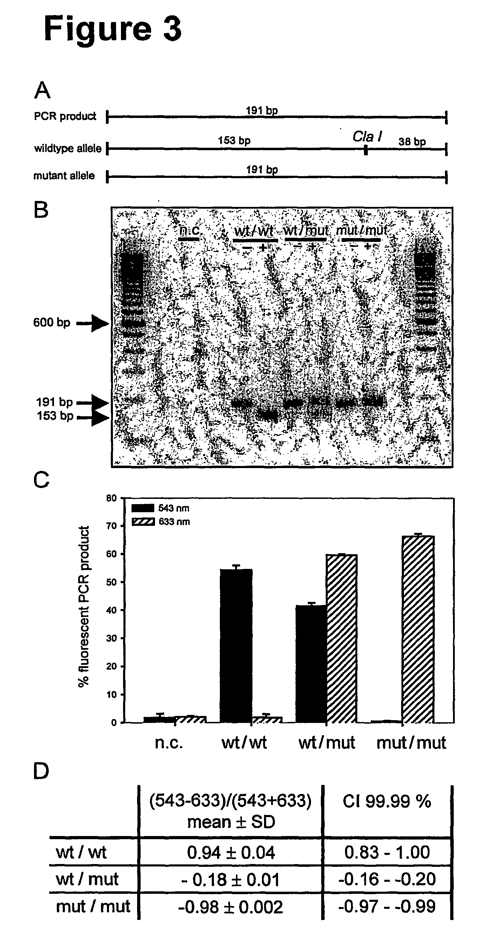

The applicability of the method of the invention for determining the presence or absence of variant nucleotides was examined by comparing it with an established method for genotyping. A sample of genomic target nucleic acid sequences with known allelic constitutions at the $A_{655}G$ polymorphic site (i.e. the variant nucleotide) of the hMLH1 gene (wt/wt: A/A; wt/mut: A/G; mut/mut: G/G) was examined in parallel by (1) conventional PCR amplification and subsequent allele-specific restriction analysis (PCR-ASRA) and by (2) the method of invention. For PCR-ASRA, a 191 bp region containing the $A_{655}G$ polymorphism of the hMLH1 gene was amplified from the target nucleic acid sequences and was subsequently digested with *ClaI* (Figure 3A, B).
By introduction of an incomplete *ClaI* restriction site at the 3'-end of the antisense amplification primer, the amplification product obtained from wildtype target nucleic acid sequences was cut into a 38 bp and a 153 bp fragment, while in the case of mutant target nucleic acid sequences, the amplification product remained undigested.
*ClaI* digestion of the amplification product obtained from heterozygous target nucleic acid sequences yielded three DNA fragments of 38 bp, 153 bp and 191 bp.
For genotyping by the method of the present invention, the same samples of genomic target nucleic acid sequences and a negative control (containing no target nucleic acid sequences) were amplified in duplicates. After thermal cycling, the reaction mixes were analyzed without any post-PCR processing, physical separation, or dilution steps by fluorescence correlation spectroscopy (Figure 3C). All samples were measured 3 times, for 3 seconds each, at 543 nm and 633 nm. The volume of the detection field at 543 nm and 633 nm was determined by measuring 5 nM TAMRA and 5 nM EVOblue™50, respectively. The translational diffusion time of the free diagnostic primers, $\tau_{free}$, defined from the negative control reaction was 0.110 ms at 543 nm and 0.103 ms at 633 nm. The translational diffusion time of the 327 bp fluorescent amplification product, $\tau_{poly}$, was automatically evaluated by curve fitting of the autocorrelation data of heterozygous (wt/mut) samples with fixed values for S and $\tau_{free}$ and yielded 0.795 ms at 543 nm and 0.911 ms at 633 nm. Because the translational diffusion times of non-extended primers and amplified products were clearly distinguishable at both 543 nm and 633 nm, the fractions of the amplified products were automatically quantified by fitting all autocorrelation curves with fixed values of S, $\tau_{free}$, and $\tau_{poly}$. Means of the relative fractions of fluorescent amplification product were calculated for each genotype.

The reactions containing hMLH1 wildtype target nucleic acid sequences produced 54.35 % amplification product at 543 nm, but only 1.85 % amplification product at 633 nm. Heterozygous target nucleic acid sequences for the hMLH1 $A_{655}G$ polymorphism yielded significant amplification product fractions at both wavelengths, 41.55 % at 543 nm and 59.60 % at 633 nm. Target nucleic acid sequences containing only the mutant hMLH1 allele had amplification product values of only 0.55 % at 543 nm, but 66.35 % at 633 nm. The control reactions which contained no target nucleic acid sequences had only very low background amplification product fractions at both wavelengths: $1.80 \pm 0.11$ % at 543 nm and % at 633 nm. In all cases, amplification product values indicating allele-specific primer extension were at least 15-fold higher than the corresponding background noise at the respective wavelength. By calculation of the ((543 nm - 633 nm)/(543 nm + 633 nm)) values from the amplification product values from each sample, representative values for each genotype (Figure 3D) were obtained. Overall, the genotyping results obtained by the method of the invention were identical to the results obtained by the PCR-ASRA genotyping procedure.

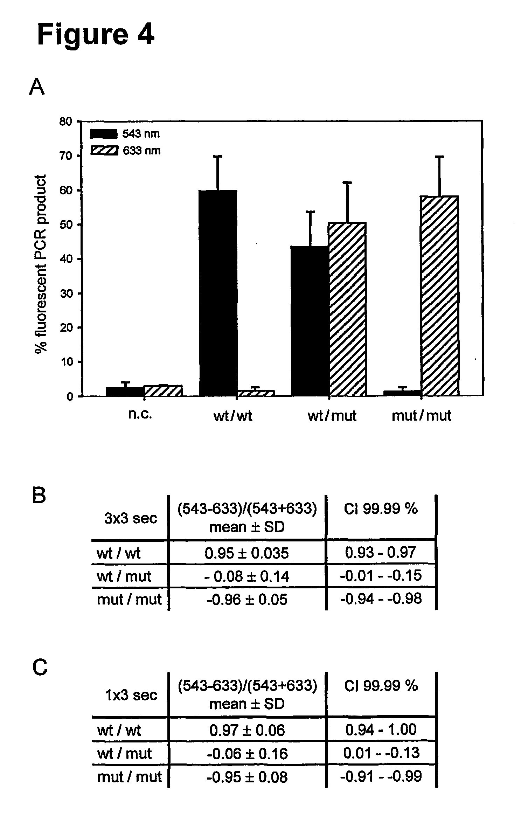

To further evaluate the specificity, reproducibility and precision of the method of invention, 18 samples of target nucleic acid sequences, (six samples for each genotype) were analyzed in duplicates by two different experimentators on three subsequent days. The total test set comprised 216 individual reactions, each individual sample of target nucleic acid sequences was genotyped 12 times, and each of the three different hMLH1 genotypes was examined in 72 independent experiments. All of the reactions resulted in correct genotype determination. These results underline the very high reproducibility (100 %) of genotyping by the method of the present invention. Figure 4B lists the mean ((543 nm - 633 nm)/(543 nm + 633 nm)) values $\pm$ SD for each genotype. With 99.99 % confidence, the values for hMLH1 wildtype, heterozygous and mutant samples range from 0.93 to 0.97, 0.01 to -0.13 and -0.91 to - 0.99, respectively. When the values for fluorescent amplification products were calculated from single FCS measurements per sample (1x 3sec at 543 nm and 633 nm), the genotypes were discriminated with the same performance (Figure 4 C).

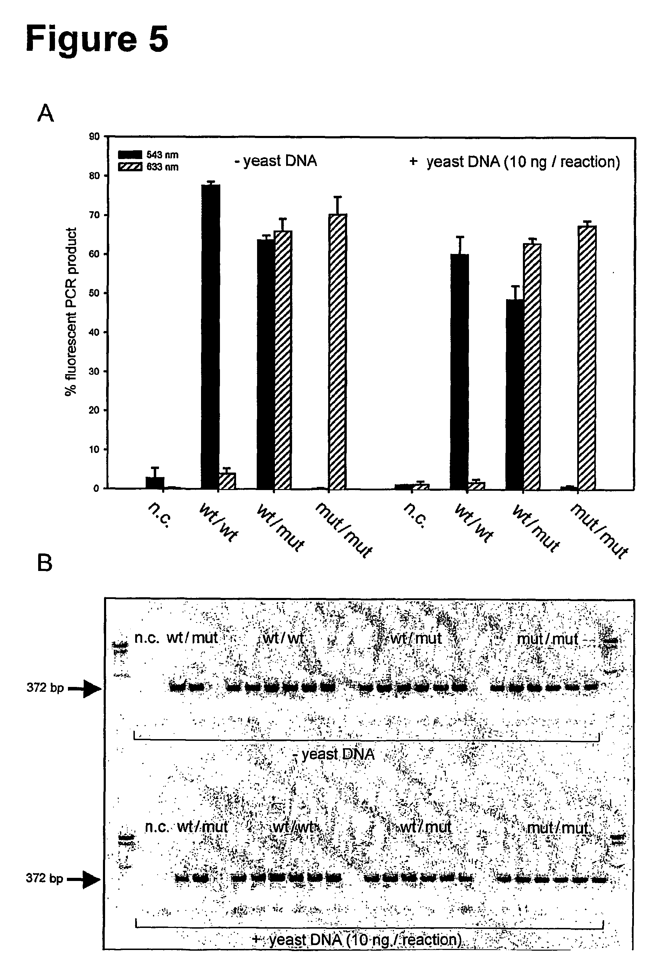

These data show that although the assays were carried out on different days, by different experimentators, and with different samples of target nucleic acid sequences for a given genotype, the obtained results were highly reproducible and precise. Furthermore, the specificity of the method of invention was examined by analyzing 9 genomic samples of target nucleic acid sequences, 3 samples for each hMLH1 genotype, in duplicates in the presence of a ~250-fold molar excess of genomic DNA from *Saccharomyces cerevisiae* (Figure 5). The genotyping results were correct in all cases and yielded values practically identical to those obtained from control reactions without *S. cerevisiae* DNA. The data demonstrated that the method of the present invention is highly specific and provides unambiguous results even in the presence of a high background of contaminating DNA.

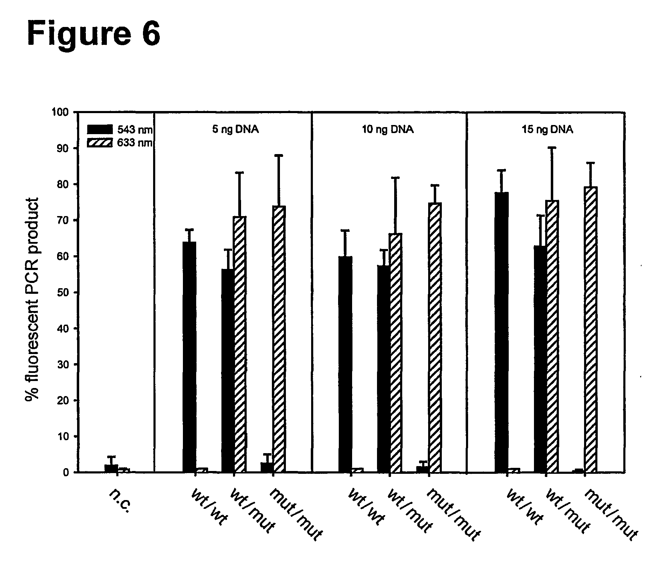

In another set of experiments, the robustness of genotyping by the method of the present invention upon addition of varying amounts of target nucleic acid sequences was examined. Three samples for each hMLH1 genotype were analyzed by the method of instant invention. The amount of target nucleic acid sequences varied from 5 to 15 ng per assay. As shown in Figure 6, changing the total amount of target nucleic acid sequences by $\pm$ 50 % did not have significant effects on the genotyping performance. Using the method of the present invention for genotyping with chimeric diagnostic primers and 5'-tail specific labeling primers, duplicate samples of genomic DNA for each hMLH1 genotype and a negative control (containing no DNA) were assayed in the presence of a set of chimeric diagnostic primers (CD1 and CD2) and the corresponding fluorescence-tagged, 5'-tail specific labeling primers (L1 and L2). In addition to the "no-DNA" negative controls, duplicate samples of genomic DNA heterozygous for the hMLH1 genotype (wt/mut) and samples containing no chimeric diagnostic primers in the presence of fluorescence-tagged, 5'-tail specific labeling primers (L1 and L2) were amplified.

After thermal cycling, the reaction mixes were analyzed by fluorescence correlation spectroscopy and, additionally, by agarose gel electrophoresis (Figure 8). For FCS analysis, all samples were measured 3 times, for 3 seconds each, at 543 nm and 633 nm. Means of the relative fractions of fluorescent amplification product were calculated for each genotype (Figure 8A). The reactions containing hMLH1 wildtype target nucleic acid sequences produced 64.35 % fluorescent amplification product at 543 nm, but only 1.75 at 633 nm. Heterozygous target nucleic acid sequences for the hMLH1 A655G polymorphism yielded significant fluorescent amplification product fractions at both wavelengths, 58.30 % at 543 nm and 53.30 % at 633 nm. Target nucleic acid sequences containing only the mutant hMLH1 allele had fluorescent amplification product values of only 5.05 % at 543 nm, but 71.20 % at 633 nm. The control reactions that contained no target nucleic acid sequences had very low fractions of fluorescent amplification product at both wavelengths (below 4 %), irrespective of being carried out with or without chimeric diagnostic primers. The control reactions that contained genomic DNA heterozygous for the hMLH1 genotype (wt/mut) but no chimeric diagnostic primers were amplified to the same extend than the other DNA-containing reactions (Figure 8B), but showed very low fractions of fluorescent amplification product at both wavelengths, comparable to the "no-DNA" negative controls. By calculation of the ((543 nm - 633 nm)/(543 nm + 633 nm)) values from the amplification product values of each sample, representative values for each genotype (Figure 8C) were obtained.

The data demonstrate that the preferred embodiment of the method as described in the present invention, using chimeric diagnostic primers and fluorescence-tagged 5'-tail specific labeling primers provides highly specific genotyping results.

Description of Figures:

Figure 1 illustrates one embodiment of the principle of the method described in the present invention for the analysis of variant nucleic acid sequences using fluorescence-labeled diagnostic primers. The gene specific amplification primers (F, R) amplify wildtype as well as mutant alleles with equal efficiency. Allele-specific diagnostic primers (D 1,D2) are attached to different and distinguishable fluorescent tags (star and closed circle, respectively) and simultaneously amplify efficiently only the allele with the corresponding variant nucleotide. This results in allele-specific accumulation of fluorescent amplification products detectable preferably by fluorescence correlation spectroscopy. When only allele A is present in the reaction, fluorescent amplification products are generated from primer D1 but not from D2. Likewise, when only allele B is present, fluorescent amplification products are generated from primer D2 but not from D1. When both allele A and allele B are present, fluorescent amplification products are generated from both primer D 1 and D2.

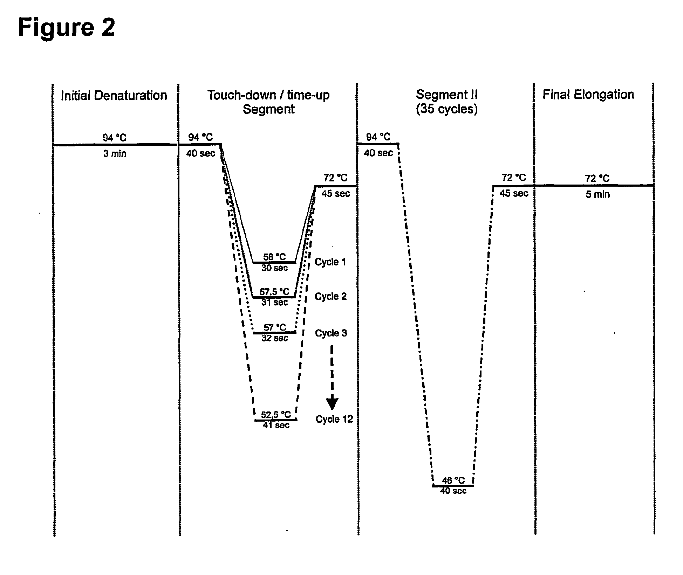

Figure 2 illustrates the thermal cycling profile applied for the method as described in the present invention.

Figure 3 illustrates the analysis of the $A_{655}G$ polymorphism of the hMLH1 gene by conventional amplification followed by allele-specific restriction analysis (PCR-ASRA) and, in comparison, by the method of the present invention using fluorescence-labeled diagnostic primers. A) Schematic illustration of the *ClaI* restriction digestion. A 191 bp amplification product from the wildtype allele is cut into a 38 bp and a 153 bp fragment, while the amplification product obtained from the mutant allele remains undigested. B) Analysis of *ClaI* restriction by agarose gel electrophoresis. Outer Lanes: 100 bp DNA ladder. Lanes containing PCR products obtained from genomic DNA representing the homozygous wildtype genotype (wt/wt), the heterozygous genotype (wt/mut), or the homozygous mutant genotype (mut/mut) for the hMLH1 $A_{655}G$ polymorphism, respectively, before (-) and after (+) *ClaI* digestion are indicated. Lane "n.c." indicates a PCR negative control (without template, i.e. "no-DNA" control). All samples yielded the expected restriction fragment patterns, i.e., 153 bp and 38 bp for the wildtype, 191 bp, 153 bp and 38 bp for the heterozygous and 191 bp for the mutant DNA sample, respectively. The 38-bp fragment being hardly visible on the gel. C) Depicts the read out of the analysis of the hMLH1 $A_{655}G$ polymorphism using fluorescence correlation spectroscopy (FCS). The relative amounts of the two fluorescence-labeled diagnostic primers and their extension products are determined by the herein presented method. The three hMLH1 genotypes (wt/wt, wt/mut, mut/mut) and a negative control without template (n.c.) are represented on the x-axis, and the corresponding amount of extension products (fluorescence signal in relative units) are shown on the y-axis. The black bars show the TAMRA-labeled products (wt) (excited at 543 nm), and the hatched bars show the EVOblue 50-labeled products (mut) (excited at 633 nm) amplified in the same reaction. D) For the purpose of more convenient genotyping, the measured fluorescence signals for the EVOblue-50 labeled PCR-products were subtracted from the the measured fluorescence signals for the TAMRA-labeled PCR-products, and the residual was divided by the sum of the measured fluorescence signals of both TAMRA- and EVOblue-50 labeled PCR-products ((543 nm - 633 nm)/(543 nm + 633 nm)). The obtained values allow for unambiguous and highly specific genotyping.

Figure 4 shows data demonstrating the specificity, reproducibility, and precision of the method of the present invention using fluorescence-labeled diagnostic primers. A) Six genomic DNA samples for each hMLH1 genotype, wt/wt, wt/mut and mut/mut, respectively, were analyzed in duplicates on three different days by two different experimentators. The graph shows means +/- SD of relative fractions of fluorescent amplification products obtained at 543 nm and at 633 nm for each genotype. B) and C) Mean ((543 nm - 633 nm)/(543 nm + 633 nm)) values +/- SD for each hMLH1 genotype are listed. Data were obtained from n = 72 independent determinations of each genotype by repetitive (3 x 3 sec; (B)) or single (1 x 3 sec; (C)) FCS measurements at 543 nm and 633 nm.

Figure 5 depicts the specificity of the method of the present invention using fluorescence-labeled diagnostic primers in the presence of contaminating template. Genotyping was performed in parallel with human genomic DNA and with a mixture of human genomic DNA and contaminating *S. cerevisiae* DNA (10 ng each). This represents a 250-fold molar excess of *S. cerevisiae* DNA over human genomic DNA. Three genomic DNA samples for each genotype were tested in duplicates. A) Data represent means +/- SD of fluorescent signals for each genotype. B) Corresponding 3 % TAE-agarose-gel electrophoresis with individual reactions.

EP 1 474 529 B1

Figure 6 exemplifies the robustness of the method of the present invention of determining the presence or absence of variant nucleotides using fluorescence-labeled diagnostic primers. The amount of target nucleic acid sequences in the assay was varied by +/- 50 % of the standard concentration (10 ng). Three genomic DNA samples for each genotype were analyzed in duplicates. Data represent means +/- SD of fluorescent signals for each genotype.

Figure 7 illustrates a preferred embodiment of the method of the present invention using chimeric diagnostic primers in conjunction with fluorescence-tagged 5'-tail specific labeling primers. Gene specific amplification primers (F, R) amplify wildtype as well as mutant alleles with equal efficiency. Allele-specific chimeric diagnostic primers (CD1, CD2) contain different and distinguishable nucleotide sequence tags, i.e. 5'-tail sequence portions, and simultaneously amplify efficiently only the allele with the corresponding variant nucleotide. This results in allele-specific accumulation of amplification products containing different and distinguishable 5'-tail sequence portions at one end. Upon further cycles of amplification, said 5'-tail sequence portions constitute priming sites for the fluorescence-tagged 5'-tail specific labeling primers (L1, L2), resulting in allele-specific accumulation of detectable fluorescent amplification products, preferably detectable by fluorescence correlation spectroscopy.

Figure 8 illustrates the analysis of the $A_{655}G$ polymorphism of the hMLH1 gene by a preferred embodiment of the method of the present invention using chimeric diagnostic primers CD1 and CD2, and fluorescence-tagged 5'-tail specific labeling primers L1 and L2. A) Duplicate genomic DNA samples for the three hMLH1 genotypes (wt/wt; wt/mut; mut/mut) and negative controls (n.c.) were analyzed in the presence (+) or absence (-) of chimeric diagnostic primers (CD1, CD2). The graph shows means +/- SD of relative fractions of fluorescent amplification products obtained at 543 nm (black bars) and 633 nm (hatched bars) for each genotype. B) Corresponding agarose gel electrophoresis of amplified reaction mixes. C) Mean (543 nm - 633 nm)/ (543 nm + 633 nm)-values +/- SD for each hMLH1 genotype are listed. Data were obtained from 18 individual (543 nm - 633 nm)/(543 nm + 633 nm)-values generated for each genotype by using all possible combinations of relative fractions of fluorescent amplification products obtained from single measurements at both wavelengths (3 measurements/ sample at 543 nm x 3 measurements/ sample at 633 nm = 9 values/ sample = 18 values/ genotype).

Figure 9 depicts a preferred embodiment of the method of the present invention when applied in a non-homogeneous format. - Step 1- upper panel: Assay setup; one primer of a pair of amplification primers (F, R) is immobilized on a solid surface, whereas the other amplification primer of said pair of amplification primers, the corresponding pair of nested diagnostic primers (D1, D2) and the nucleic acid template to be analyzed are in the liquid phase. - Step 2 - middle panel: Different types of free and immobilized primers and extended products after amplification. - Step 3 - lower panel: Different types of immobilized amplification products after subsequent stringent washing procedure.

**Claims**

1. A method of detecting the presence or absence of variant nucleotides within target nucleic acid sequences, said method comprising the following steps:

   (i) setting up a reaction mix comprising:

   • nucleoside triphosphates, or functional derivatives thereof
   • a polymerizing agent
   • at least one pair of target-specific amplification primers capable of hybridization to target nucleic acid sequences
   • at least one set of diagnostic primers, each set consisting of at least two types of diagnostic primers capable of variable allele-specific hybridization to corresponding target nucleic acid sequences 3'-relative to said amplification primers, such that each set of diagnostic primers is semi-nested relative to the corresponding pair of amplification primers,

the at least two types of diagnostic primers of a set of diagnostic primers being **characterized by** having substantially similar nucleotide sequences except for at least one internally located nucleotide, which is different for each type of diagnostic primer and complementary to the variant nucleotide, whereby an extended diagnostic primer is being synthesized if the internally located nucleotide of the diagnostic primer is complementary to the corresponding nucleotide in the target nucleic acid sequence, and whereby substantially no or a negligible background of extended diagnostic primer is being synthesized if said internal nucleotide is not complementary to the corresponding nucleotide in the target nucleic acid sequence, and each type of diagnostic primer further being **characterized by** carrying a different and distinguishable tag; and

• at least one sample of target nucleic acid sequences

(ii) performing an amplification reaction under conditions permitting hybridization of the amplification primers and variable allele specific hybridization of the diagnostic primers, either together or sequentially, to the corresponding target nucleic acid sequences, and promoting polymerization; and

(iii) monitoring specific properties of said types of diagnostic primers during or after completion of the amplification reaction, said specific properties being indicative for an extension or non-extension of the diagnostic primers, thereby detecting the presence or absence of a variant nucleotide contained within a target nucleic acid sequence.

2. The method according to claim 1, wherein the concentration of each diagnostic primer is 0.5 to 0.001 times, preferably 0.25 to 0.01 times, most preferably 0,1 to 0.003 times the concentration of each amplification primer.

3. The method according to claims 1 and 2, wherein the diagnostic primers comprise a sequence of about 5 to 100 nucleotides, preferably 5 to 50 nucleotides, most preferably 5 to 40 nucleotides in length.

4. The method according to claims 1 to 3 wherein each diagnostic primer contains at least one tag selected from the group consisting of fluorescent dyes, chemiluminescent tags, electroluminescent tags, magnetic tags, affinity or binding tags, nucleotide sequence tags, position specific tags, and/or tags with specific physical properties such as different size, mass, gyration, ionic strength, dielectric properties, polarization, or impedance.

5. The method according to claims 1 to 4 wherein the diagnostic primers contain fluorescent dyes, and wherein each type of diagnostic primer is tagged with a different fluorescent dye, such that each type of diagnostic primer is **characterized by** different excitation- and/or emission spectra, life-time properties, polarization properties, fluorescence-resonance-energy-transfer (FRET) properties, quantum yields, photostability, or triplet number of fluorochromes.

6. The method according to claims 1 to 5 wherein the tag is permanently or temporarily attached to the diagnostic primer, preferably to the 5'- end of the diagnostic primer.

7. The method according to claims 1 to 5 wherein a diagnostic primer is a chimeric diagnostic primer, and wherein each chimeric diagnostic primer comprises a different and distinguishable 5'-tail permanently attached to the 5'-end of the diagnostic primer, and wherein the diagnostic primer portion of the chimeric diagnostic primer is complementary to the target nucleotide sequence in a variant nucleotide specific manner, and wherein the 5'-tail portion is non-complementary to the target nucleotide sequence.

8. The method according to claim 7 wherein the presence or absence of each of the different and distinguishable 5'-tails is detected with a corresponding type of 5'-tail specific probe.

9. The method according to claim 8 wherein said 5'-tail specific probe is a 5'-tail specific labeling primer and/or a 5'-tail specific hybridization probe.

10. The method according to claims 8 to 9 wherein said 5'-tail specific probe contains at least one tag selected from the group consisting of fluorescent dyes, chemiluminescent tags, electroluminescent tags, magnetic tags, affinity or binding tags, nucleotide sequence tags, position specific tags, and/or tags with specific physical properties such as different size, mass, gyration, ionic strength, dielectric properties, polarization, or impedance.

11. The method according to claim 10 wherein said 5'-tail specific probe contains fluorescent dyes, and wherein each type of 5'-tail specific probe is tagged with a different fluorescent dye, such that each type of 5'-tail specific probe is **characterized by** different excitation- and/or emission spectra, life-time properties, polarization properties, fluorescence-resonance-energy-transfer (FRET) properties, quantum yields, photostability, or triplet number of fluorochromes.

12. The method according to claims 10 and 11 wherein the tag is permanently or temporarily attached to the 5'-tail specific probe, preferably to the 5'- end of the 5'-tail specific probe.

13. The method according to claims 1 to 12 wherein the amplification reaction and the monitoring of specific properties are performed in a homogeneous format.

14. The method according to claims 1 to 13 wherein at least one of the amplification primers is immobilized, preferably at its 5'-terminus, to a solid support.

15. The method according to claims 1 to 14 wherein the polymerizing agent is an enzyme, preferably a DNA polymerase, preferably a thermostable DNA polymerase, most preferably a thermostable DNA polymerase which lacks 5'-to 3'-exonuclease activity.

16. The method according to claims 1 to 15 wherein a sample of target nucleic acid sequences is selected from a group comprising genomic DNA, cDNA, single stranded DNA, double stranded DNA, plasmid DNA, RNA, mixtures of DNA with other molecules, DNA or RNA from human sources or other sources such as mammals, vertebrates, invertebrates, bacteria, viruses, yeast, fungi, or plants.

17. The method according to claims 1 to 16 wherein the monitoring of the specific properties of said amplification reaction products is determined by chromatographic techniques such as HPLC, FPLC, capillary electrophoresis, gel electrophoresis, or by mass spectroscopic or electrochemical techniques or, preferably by fluorescent techniques such as fluorescent polarization spectroscopy, fluorescent life-time spectroscopy' fluorescence intensity distribution analysis (FIDA), fluorescence dichroism analysis, fluorescence intensity analysis, fluorescence resonance energy transfer (FRET) analysis, spectroscopic analysis of excitation and/or emission spectra, or, in particular, by fluorescence correlation spectroscopy.

18. The According to claims 1 to 17 wherein monitoring the degree of extension of a diagnostic primer or a chimeric diagnostic primer comprises the qualitative detection of variant nulceotides contained within a sample of target nucleic acid sequences, insofar as an extended diagnostic primer or chimeric diagnostic primer is indicative for the pretence of' a variant nucleotide and a non-extended diagnostic primer or chimeric diagnostic primer is indicative for the absence of a variant nucleotide.

19. The method according to claims 1 to 18 wherein motoring the amounts of extended diagnostic primers or chimeric diagnostic primers comprises the quantification of target nucleic acid sequences, in particular the genotyping of samples of target nucleic acid sequences, preferably by (i) calculating the ratio of the values of individually recorded signals, or (ii) by subtracting the value of signal B from the value of signal A, or (iii) by dividing the value of the remainder of signal A after subtraction of signal B by the value of the sum of signal A and signal B.

20. Use of the method according to claims 1 to 19 for detecting the presence or absence of one or more variant nucleotides in a sample of target nucleic acid sequences.

21. Use of the method according to claim 20 for diagnostic screening of a sample of target nucleic acid sequences for inherited or acquired conditions and diseases, and for screening for predispositons to such conditions and diseases.

22. Use of the method according, to claims 1 to 19 for quantifying target nucleic acid sequences, in particular for genotyping target nucleic acid sequences, determining the concentration of DNA, or RNA, or mRNA, or viral RNA in a sample, or determining the copy-number of target nucleic acid sequences, particularly determining the copy-number of target nucleic acid sequences in samples derived from tumor tissue.

23. Use of the method according to claims 1 to 19 for assaying the methylation status, preferably the status of cytosine methylation, of target nucleic acid sequences.

**24.** Use of a kit for detecting the presence or absence of variant nucleotides within a sample of target nucleic acid sequences in a method according to claims 1-19, said kit comprising in packaged combination:

- nucleoside triphosphates or functional derivatives thereof
- a polymerizing agent
- at least one pair of target-specific amplification primers capable of hybridization to target nucleic acid sequences
- at least one set of diagnostic primers, each set consisting of at least two types of diagnostic primers capable of variable allele-specific hybridization to corresponding target nucleic acid sequences 3'-relative to said amplification primers, such that each set of diagnostic primers is semi-nested relative to the corresponding pair or amplification primers,

the at least two types of diagnostic primers of a set of diagnostic primers being **characterized by** having substantially similar nucleotide sequences except for at least one internally located nucleotide, which is different for each type of diagnostic primer and complementary to the variant nucleotide, whereby an extended diagnostic primer is being synthesized if the internally located nucleotide of the diagnostic primer is complementary to the corresponding nucleotide in the targets nucleic acid sequence, and whereby substantially no or a negligible background of extended diagnostic primer is being synthesized if said internal nucleotide is not complementary to the corresponding nucleotide in the target nucleic acid sequence,

and each type of diagnostic primer further being **characterized by** carrying a different and distinguishable tag.

**25.** Use of a kit according to claim 24 wherein said diagnostic primer is a chimeric diagnostic primer.

**26.** Use of a kit according to claim 25 further comprising 5'-tail specific probes.

**27.** Use of a kit according to according to claims 24 to 26 comprising at least one sample of internal standard nucleic acid sequences.

**28.** Use of a kit according to claims 24 to 27 for detecting the presence or absence of one or more variant nucleotides associated with an inherited or acquired condition or disease, for genotyping, for quantification of target nucleic acid sequences, and for assaying the methylation status, preferably the status of cytosine methylation of target nucleic acid sequences.


**Patentansprüche**

**1.** Verfahren zum Nachweisen der Anwesenheit oder Abwesenheit von abweichenden Nucleotiden innerhalb von Zielnucleinsäuresequenzen, wobei das Verfahren die folgenden Schritte umfasst:

(i) Herstellen eines Reaktionsgemischs, umfassend:

- Nucleosidtriphosphate oder funktionelle Derivate davon;
- ein Polymerisationsmittel;
- wenigstens ein Paar von zielspezifischen Amplifikationsprimern, die mit Zielnucleinsäuresequenzen hybridisieren können;
- wenigstens ein Satz diagnostischer Primer, wobei jeder Satz aus wenigstens zwei Typen von diagnostischen Primern besteht, die zur variablen allelspezifischen Hybridisierung mit entsprechenden Zielnucleinsäuresequenzen 3' von den Amplifikationsprimern befähigt sind, so dass jeder Satz diagnostischer Primer relativ zu dem entsprechenden Paar von Amplifikationsprimern halbgeschachtelt (semi-nested) ist;

wobei wenigstens zwei Typen von diagnostischen Primern eines Satzes von diagnostischen Primern **dadurch gekennzeichnet sind, dass** sie im Wesentlichen ähnliche Nucleotidsequenzen haben, abgesehen von wenigstens einem intern lokalisierten Nucleotid, das für jeden Typ von diagnostischem Primer verschieden und zu dem abweichenden Nucleotid komplementär ist, wodurch ein verlängerter diagnostischer Primer synthetisiert wird, wenn das intern lokalisierte Nucleotid des diagnostischen Primers komplementär zu dem entsprechenden Nucleotid in der Zielnucleinsäuresequenz ist, und wodurch im Wesentlichen kein oder nur ein vernachlässigbarer Hintergrund von verlängertem diagnostischen Primer synthetisiert wird, wenn das interne Nucleotid nicht komplementär zu dem entsprechenden Nucleotid in der Zielnucleinsäuresequenz ist, und jeder Typ von diagnostischem Primer weiterhin **dadurch gekennzeichnet ist, dass** er eine andere und unterscheidbare Markierung trägt; und

• wenigstens eine Probe von Zielnucleinsäuresequenzen;

(ii) Durchführen einer Amplifikationsreaktion unter Bedingungen, die die Hybridisierung der Amplifikationsprimer und die variable allelspezifische Hybridisierung der diagnostischen Primer, entweder zusammen oder nacheinander, an die entsprechenden Zielnucleinsäuresequenzen ermöglichen, und Fördern der Polymerisation; und
(iii) Messen spezieller Eigenschaften der Typen von diagnostischen Primern während oder nach der Beendigung der Amplifikationsreaktion, wobei die spezifischen Eigenschaften auf eine Verlängerung oder Nichtverlängerung der diagnostischen Primer hinweisen, wodurch die Anwesenheit oder Abwesenheit eines abweichenden Nucleotids innerhalb einer Zielnucleinsäuresequenz nachgewiesen wird.

2. Verfahren gemäß Anspruch 1, wobei die Konzentration jedes diagnostischen Primers das 0,5- bis 0,001-fache, vorzugsweise das 0,25- bis 0,01-fache, am meisten bevorzugt das 0,1- bis 0,003-fache, der Konzentration jedes Amplifikationsprimers beträgt.

3. Verfahren gemäß Anspruch 1 und 2, wobei die diagnostischen Primer eine Sequenz mit einer Länge von etwa 5 bis 100 Nucleotiden, vorzugsweise 5 bis 50 Nucleotiden, am meisten bevorzugt 5 bis 40 Nucleotiden umfassen.

4. Verfahren gemäß Anspruch 1 bis 3, wobei jeder diagnostische Primer wenigstens eine Markierung enthält, die aus der Gruppe ausgewählt ist, die aus Fluoreszenzfarbstoffen, Chemilumineszenzmarkern, Elektrolumineszenzmarkern, magnetischen Markern, Affinitäts- oder Bindungsmarkern, Nucleotidsequenzmarkern, positionsspezifischen Markern und/oder Markern mit speziellen physikalischen Eigenschaften, wie unterschiedlicher Größe, Masse, optischer Aktivität, Ionenstärke, dielektrischen Eigenschaften, Polarisation oder Impedanz, besteht.

5. Verfahren gemäß Anspruch 1 bis 4, wobei die diagnostischen Primer Fluoreszenzfarbstoffe enthalten und wobei jeder Typ von diagnostischem Primer mit einem anderen Fluoreszenzfarbstoff markiert ist, so dass jeder Typ von diagnostischem Primer durch unterschiedliche Anregungs- und/oder Emissionsspektren, Lebensdauereigenschaften, Polarisationseigenschaften, Fluoreszenz-Resonanz-Energie-Transfer(FRET)-Eigenschaften, Quantenausbeuten, Photostabilität oder Triplettzahl der Fluorochrome **gekennzeichnet** ist.

6. Verfahren gemäß Anspruch 1 bis 5, wobei die Markierung permanent oder temporär an den diagnostischen Primer, vorzugsweise das 5'-Ende des diagnostischen Primers, gebunden ist.

7. Verfahren gemäß Anspruch 1 bis 5, wobei ein diagnostischer Primer ein chimärischer diagnostischer Primer ist und wobei jeder chimärische diagnostische Primer einen anderen und unterscheidbaren 5'-Schwanz umfasst, der permanent an das 5'-Ende des diagnostischen Primers gebunden ist, und wobei der diagnostische-Primer-Teil des chimärischen diagnostischen Primers in varianter nucleotidspezifischer Weise komplementär zur Zielnucleotidsequenz ist und wobei der 5'-Schwanzteil nicht komplementär zur Zielnucleotidsequenz ist.

8. Verfahren gemäß Anspruch 7, wobei die An- oder Abwesenheit jedes der anderen und unterscheidbaren 5'-Schwänze mit einer entsprechenden Art von 5'-Schwanz-spezifischer Sonde nachgewiesen wird.

9. Verfahren gemäß Anspruch 8, wobei die 5'-Schwanz-spezifische Sonde ein 5'-Schwanz-spezifischer Markierungsprimer und/oder eine 5'-Schwanz-spezifische Hybridisierungssonde ist.

10. Verfahren gemäß Anspruch 8 bis 9, wobei die 5'-Schwanz-spezifische Sonde wenigstens eine Markierung enthält, die aus der Gruppe ausgewählt ist, die aus Fluoreszenzfarbstoffen, Chemilumineszenzmarkern, Elektrolumineszenzmarkern, magnetischen Markern, Affinitäts- oder Bindungsmarkern, Nucleotidsequenzmarkern, positionsspezifischen Markern und/oder Markern mit speziellen physikalischen Eigenschaften, wie unterschiedlicher Größe, Masse, optischer Aktivität, Ionenstärke, dielektrischen Eigenschaften, Polarisation oder Impedanz, besteht.

11. Verfahren gemäß Anspruch 10, wobei die 5'-Schwanz-spezifische Sonde Fluoreszenzfarbstoffe enthält und wobei jeder Typ von 5'-Schwanz-spezifischer Sonde mit einem anderen Fluoreszenzfarbstoff markiert ist, so dass jeder Typ von 5'-Schwanz-spezifischer Sonde durch unterschiedliche Anregungs- und/oder Emissionsspektren, Lebensdauereigenschaften, Polarisationseigenschaften, Fluoreszenz-Resonanz-Energie-Transfer(FRET)-Eigenschaften, Quantenausbeuten, Photostabilität oder Triplettzahl der Fluorochrome **gekennzeichnet** ist.

12. Verfahren gemäß Anspruch 10 und 11, wobei die Markierung permanent oder temporär an die 5'-Schwanz-spezifische Sonde, vorzugsweise das 5'-Ende der 5'-Schwanz-spezifischen Sonde, gebunden ist.

13. Verfahren gemäß Anspruch 1 bis 12, wobei die Amplifikationsreaktion und das Messen spezieller Eigenschaften in einem homogenen Format durchgeführt werden.

14. Verfahren gemäß den Ansprüchen 1 bis 13, wobei wenigstens einer der Amplifikationsprimer an einem festen Träger immobilisiert ist, vorzugsweise an seinem 5'-Terminus.

15. Verfahren gemäß den Ansprüchen 1 bis 14, wobei das Polymerisationsmittel ein Enzym ist, vorzugsweise eine DNA-Polymerase, vorzugsweise eine thermostabile DNA-Polymerase, am meisten bevorzugt eine thermostabile DNA-Polymerase, der die 5'→3'-Exonudease-Aktivität fehlt.

16. Verfahren gemäß den Ansprüchen 1 bis 15, wobei die Probe von Zielnucleinsäuresequenzen aus einer Gruppe ausgewählt ist, die aus genomischer DNA, cDNA, einzelsträngiger DNA, doppelsträngiger DNA, Plasmid-DNA, RNA, Gemischen von DNA mit anderen Molekülen, DNA oder RNA aus menschlichen Quellen oder anderen Quellen, wie Säugern, Wirbeltieren, Wirbellosen, Bakterien, Viren, Hefen, Pilzen oder Pflanzen, besteht.

17. Verfahren gemäß den Ansprüchen 1 bis 16, wobei das Messen der speziellen Eigenschaften der Amplifikationsreaktionsprodukte durch chromatographische Techniken, wie HPLC, FPLC, Kapillarelektrophorese, Gelelektrophorese, oder durch massenspektroskopische oder elektrochemische Techniken oder vorzugsweise durch Fluores-zenztechniken, wie Fluoreszenzpolarisationsspektroskopie, Fluoreszenzlebensdauerspektroskopie, Fluoreszen-zintensitätsverteilungsanalyse (FIDA), Fluoreszenzdichroismusanalyse, Fluoreszenzintensitätsanalyse, Fluores-zenz-Resonanz-EnergieTransfer(FRET)-Analyse, spektroskopische Analyse von Anregungs- und/oder Emissionsspektren oder insbesondere durch Fluoreszenzkorrelationsspektroskopie erfolgt.

18. Verfahren gemäß den Ansprüchen 1 bis 17, wobei das Messen des Verlängerungsgrads eines diagnostischen Primers oder eines chimärischen diagnostischen Primers insofern den qualitativen Nachweis von abweichenden Nucleotiden innerhalb einer Probe von Zielnucleinsäuresequenzen umfasst, als ein verlängerter diagnostischer Primer oder chimärischer diagnostischer Primer die Anwesenheit eines abweichenden Nucleotids anzeigt und ein nicht verlängerter diagnostischer Primer oder chimärischer diagnostischer Primer die Abwesenheit eines abweichenden Nucleotids anzeigt.

19. Verfahren gemäß den Ansprüchen 1 bis 18, wobei das Messen der Menge der verlängerten diagnostischen Primer oder chimärischen diagnostischen Primer die Quantifizierung von Zielnucleinsäuresequenzen, insbesondere die Genotypbestimmung von Proben von Zielnucleinsäuresequenzen, umfasst, vorzugsweise (i) durch Berechnen des Verhältnisses der Werte von individuell aufgezeichneten Signalen oder (ii) durch Subtrahieren des Werts von Signal B von dem Wert von Signal A oder (iii) durch Dividieren des Werts der Differenz von Signal A nach Subtraktion von Signal B durch den Wert der Summe von Signal A und Signal B.

20. Verwendung des Verfahrens gemäß den Ansprüchen 1 bis 19 zum Nachweis der Anwesenheit oder Abwesenheit von einem oder mehreren abweichenden Nucleotiden in einer Probe von Zielnucleinsäuresequenzen.

21. Verwendung des Verfahrens gemäß Anspruch 20 zum diagnostischen Durchmustern von Proben von Zielnucleinsäuresequenzen auf ererbte oder erworbene Zustände und Krankheiten und zum Durchmustern auf Prädispositionen für solche Zustände und Krankheiten.

22. Verwendung des Verfahrens gemäß den Ansprüchen 1 bis 19 zum Quantifizieren von Zielnucleinsäuresequenzen, insbesondere zur Genotypbestimmung von Zielnucleinsäuresequenzen, zum Bestimmen der Konzentration von DNA oder RNA oder mRNA oder viraler RNA in einer Probe oder zum Bestimmen der Kopienzahl von Zielnucleinsäuresequenzen, insbesondere zum Bestimmen der Kopienzahl von Zielnucleinsäuresequenzen in Proben, die von Tumorgewebe abgeleitet sind.

23. Verwendung des Verfahrens gemäß den Ansprüchen 1 bis 19 zum Bestimmen des Methylierungsstatus, vorzugsweise des Status der Cytosinmethylierung, von Zielnucleinsäuresequenzen.

24. Verwendung eines Kits zum Nachweisen der Anwesenheit oder Abwesenheit von abweichenden Nucleotiden innerhalb einer Probe von Zielnucleinsäuresequenzen in einem Verfahren gemäß den Ansprüchen 1 bis 19, wobei der Kit Folgendes in Verpackungskombination umfasst:

  • Nucleosidtriphosphate oder funktionelle Derivate davon;

• ein Polymerisationsmittel;

• wenigstens ein Paar von zielspezifischen Amplifikationsprimern, die mit Zielnucleinsäuresequenzen hybridisieren können;

• wenigstens ein Satz diagnostischer Primer, wobei jeder Satz aus wenigstens zwei Typen von diagnostischen Primern besteht, die zur variablen allelspezifischen Hybridisierung mit entsprechenden Zielnucleinsäuresequenzen 3' von den Amplifikationsprimern befähigt sind, so dass jeder Satz diagnostischer Primer relativ zu dem entsprechenden Paar von Amplifikationsprimern halbgeschachtelt (semi-nested) ist;

wobei wenigstens zwei Typen von diagnostischen Primern eines Satzes von diagnostischen Primern **dadurch gekennzeichnet sind, dass** sie im Wesentlichen ähnliche Nucleotidsequenzen haben, abgesehen von wenigstens einem intern lokalisierten Nucleotid, das für jeden Typ von diagnostischem Primer verschieden und zu dem abweichenden Nucleotid komplementär ist, wodurch ein verlängerter diagnostischer Primer synthetisiert wird, wenn das intern lokalisierte Nucleotid des diagnostischen Primers komplementär zu dem entsprechenden Nucleotid in der Zielnucleinsäuresequenz ist, und wodurch im Wesentlichen kein oder nur ein vernachlässigbarer Hintergrund von verlängertem diagnostischen Primer synthetisiert wird, wenn das interne Nucleotid nicht komplementär zu dem entsprechenden Nucleotid in der Zielnucleinsäuresequenz ist, und

jeder Typ von diagnostischem Primer weiterhin **dadurch gekennzeichnet ist, dass** er eine andere und unterscheidbare Markierung trägt.

25. Verwendung eines Kits gemäß Anspruch 24, wobei der diagnostische Primer ein chimärischer diagnostischer Primer ist.

26. Verwendung eines Kits gemäß Anspruch 25, der weiterhin 5'-Schwanz-spezifische Sonden umfasst.

27. Verwendung eines Kits gemäß Anspruch 24 bis 26, der wenigstens eine Probe von internen Standardnucleinsäuresequenzen umfasst.

28. Verwendung eines Kits gemäß Anspruch 24 bis 27 zum Nachweisen der Anwesenheit oder Abwesenheit von einem oder mehreren abweichenden Nucleotiden, die mit einem ererbten oder erworbenen Zustand bzw. Krankheit zusammenhängen, zur Genotypbestimmung, zur Quantifizierung von Zielnucleinsäuresequenzen und zum Bestimmen des Methylierungsstatus, vorzugsweise des Status der Cytosinmethylierung, von Zielnucleinsäuresequenzen.


**Revendications**

1. Procédé de détection de la présence ou de l'absence de nucléotides variants dans des séquences d'acide nucléique cible, ledit procédé comprenant les étapes suivantes consistant à :

    (i) élaborer un mélange réactionnel comprenant :

        - des nucléoside-triphosphates ou des dérivés fonctionnels de ceux-ci
        - un agent polymérisant
        - au moins une paire d'amorces d'amplification spécifiques pour une cible capables de s'hybrider sur des séquences d'acide nucléique cible
        - au moins un jeu d'amorces à but diagnostique, chaque jeu étant constitué d'au moins deux types d'amorces à but diagnostique capables de s'hybrider de manière spécifique pour un allèle variable sur des séquences d'acide nucléique cible correspondantes côté 3' par rapport auxdites amorces d'amplification, de sorte que chaque jeu d'amorces à but diagnostique est semi-niché par rapport à la paire correspondante d'amorces d'amplification,
        les au moins deux types d'amorces à but diagnostique d'un jeu d'amorces à but diagnostique étant **caractérisés en ce qu'**ils ont des séquences de nucléotides essentiellement similaires à l'exception d'au moins un nucléotide interne, qui est différent pour chaque type d'amorce à but diagnostique et complémentaire du nucléotide variant, si bien qu'une amorce à but diagnostique allongée est synthétisée si le nucléotide interne de l'amorce à but diagnostique est complémentaire du nucléotide correspondant dans la séquence d'acide nucléique cible, et si bien qu'un bruit de fond essentiellement nul ou négligeable d'amorce à but diagnostique allongée est synthétisé si ledit nucléotide interne n'est pas complémentaire du nucléotide correspondant dans la séquence d'acide nucléique cible et chaque type d'amorce à but diagnostique étant en outre **caractérisé en ce qu'**il porte un marqueur différent et pouvant être différencié ; et
        - au moins un échantillon de séquences d'acide nucléique cible

(ii) réaliser une réaction d'amplification dans des conditions permettant l'hybridation des amorces d'amplification et l'hybridation spécifique pour un allèle variable des amorces à but diagnostique, soit simultanément soit successivement, sur les séquences d'acide nucléique cible correspondantes, et activer la polymérisation ; et
(iii) contrôler les propriétés spécifiques desdits types d'amorces à but diagnostique pendant ou après l'achèvement de la réaction d'amplification, lesdites propriétés spécifiques étant indicatrices d'un allongement ou d'un non-allongement des amorces à but diagnostique, détectant ainsi la présence ou l'absence d'un nucléotide variant présent dans une séquence d'acide nucléique cible.

2. Procédé selon la revendication 1, dans lequel la concentration de chaque amorce à but diagnostique est de 0,5 à 0,001 fois, de préférence de 0,25 à 0,01 fois et de manière tout particulièrement préférée de 0,1 à 0,003 fois la concentration de chaque amorce d'amplification.

3. Procédé selon les revendications 1 et 2, dans lequel les amorces à but diagnostique comprennent une séquence d'environ 5 à 100 nucléotides, de préférence 5 à 50 nucléotides, de manière tout particulièrement préférée 5 à 40 nucléotides de long.

4. Procédé selon les revendications 1 à 3, dans lequel chaque amorce à but diagnostique contient au moins un marqueur choisi dans le groupe constitué par les colorants fluorescents, les marqueurs chimioluminescents, les marqueurs électroluminescents, les marqueurs magnétiques, les marqueurs de liaison ou d'affinité, les marqueurs de type séquence de nucléotides, les marqueurs spécifiques pour une position et/ou les marqueurs ayant des caractéristiques physiques spécifiques telles qu'une taille, une masse, un rayon de giration, une force ionique, des propriétés diélectriques, une polarisation ou une impédance différentes.

5. Procédé selon les revendications 1 à 4, dans lequel les amorces à but diagnostique contiennent des colorants fluorescents et dans lequel chaque type d'amorce à but diagnostique est marqué avec un colorant fluorescent différent, de sorte que chaque type d'amorce à but diagnostique se **caractérise par** des propriétés de spectre d'excitation et/ou d'émission, des propriétés de durée de vie, des propriétés de polarisation, des propriétés de transfert d'énergie par résonance de fluorescence (FRET), des rendements quantiques, une stabilité à la lumière ou un nombre de triplets de fluorochromes différents.

6. Procédé selon les revendications 1 à 5, dans lequel le marqueur est fixé de manière permanente ou temporaire à l'amorce à but diagnostique, de préférence à l'extrémité 5' de l'amorce à but diagnostique.

7. Procédé selon les revendications 1 à 5, dans lequel une amorce à but diagnostique est une amorce à but diagnostique chimère et dans lequel chaque amorce à but diagnostique chimère comprend une queue 5' différente et pouvant être différenciée fixée de façon permanente à l'extrémité 5' de l'amorce à but diagnostique, et dans lequel la partie d'amorce à but diagnostique de l'amorce à but diagnostique chimère est complémentaire de la séquence de nucléotides cible de manière spécifique pour un nucléotide variant, et dans lequel la partie de queue 5' n'est pas complémentaire de la séquence de nucléotides cible.

8. Procédé selon la revendication 7, dans lequel la présence ou l'absence de chacune des queues 5' différentes et pouvant être différenciées est détectée à l'aide d'un type correspondant de sonde spécifique pour une queue 5'.

9. Procédé selon la revendication 8, dans lequel ladite sonde spécifique pour une queue 5' est une amorce de marquage spécifique pour une queue 5' et/ou une sonde d'hybridation spécifique pour une queue 5'.

10. Procédé selon les revendications 8 à 9, dans lequel ladite sonde spécifique pour une queue 5' contient au moins un marqueur choisi dans le groupe constitué par les colorants fluorescents, les marqueurs chimioluminescents, les marqueurs électroluminescents, les marqueurs magnétiques, les marqueurs de liaison ou d'affinité, les marqueurs de type séquence de nucléotides, les marqueurs spécifiques pour une position et/ou les marqueurs ayant des caractéristiques physiques spécifiques telles qu'une taille, une masse, un rayon de giration, une force ionique, des propriétés diélectriques, une polarisation ou une impédance différentes.

11. Procédé selon la revendication 10, dans lequel les sondes spécifiques pour une queue 5' contiennent des colorants fluorescents et dans lequel chaque type de sonde spécifique pour une queue 5' est marqué avec un colorant fluorescent différent, de sorte que chaque type de sonde spécifique pour une queue 5' se **caractérise par** des propriétés de spectre d'excitation et/ou d'émission, des propriétés de durée de vie, des propriétés de polarisation, des propriétés de transfert d'énergie par résonance de fluorescence (FRET), des rendements quantiques, une stabilité à la lumière ou un nombre de triplets de fluorochromes différents.

12. Procédé selon les revendications 10 et 11, dans lequel le marqueur est fixé de manière permanente ou temporaire à la sonde spécifique pour une queue 5', de préférence à l'extrémité 5' de la sonde spécifique pour une queue 5'.

13. Procédé selon les revendications 1 à 12, dans lequel la réaction d'amplification et le contrôle des propriétés spécifiques sont réalisés dans un système homogène.

14. Procédé selon les revendications 1 à 13, dans lequel au moins une des amorces d'amplification est immobilisée, de préférence au niveau de son extrémité 5', sur un support solide.

15. Procédé selon les revendications 1 à 14, dans lequel l'agent polymérisant est une enzyme, de préférence une ADN-polymérase, de préférence une ADN-polymérase thermostable, de manière tout particulièrement préférée une ADN-polymérase thermostable qui ne présente pas l'activité d'une 5'→3' exonucléase.

16. Procédé selon les revendications 1 à 15, dans lequel un échantillon de séquences d'acide nucléique cible est choisi dans un groupe comprenant l'ADN génomique, l'ADNc, l'ADN monocaténaire, l'ADN bicaténaire, l'ADN plasmidique, l'ARN, les mélanges d'ADN avec d'autres molécules, l'ADN ou l'ARN provenant de sources humaines ou d'autres sources telles que des mammifères, des vertébrés, des invertébrés, des bactéries, des virus, des levures, des champignons ou des végétaux.

17. Procédé selon les revendications 1 à 16, dans lequel le contrôle des propriétés spécifiques desdits produits de la réaction d'amplification est réalisé par des techniques chromatographiques telles que la CLHP, la chromatographie liquide en phase éclair FPLC, l'électrophorèse capillaire, l'électrophorèse sur gel ou par des techniques électrochimiques ou spectroscopiques de masse ou, de préférence, par des techniques de fluorescence telles que la spectroscopie de polarisation de fluorescence, la spectroscopie de durée de vie de fluorescence, l'analyse de distribution de l'intensité de fluorescence (FIDA), l'analyse du dichroïsme de fluorescence, l'analyse de l'intensité de fluorescence, l'analyse de transfert d'énergie par résonance de fluorescence (FRET), l'analyse spectroscopique du spectre d'excitation et/ou d'émission ou, en particulier, par spectroscopie à corrélation de fluorescence.

18. Procédé selon les revendications 1 à 17, dans lequel le contrôle du degré d'allongement d'une amorce à but diagnostique ou d'une amorce à but diagnostique chimère comprend la détection qualitative de nucléotides variants contenus dans un échantillon de séquences d'acide nucléique cible, dans la mesure où l'amorce à but diagnostique ou d'une amorce à but diagnostique chimère allongée est indicatrice de la présence d'un nucléotide variant et une amorce à but diagnostique ou d'une amorce à but diagnostique chimère non allongée est indicatrice de l'absence d'un nucléotide variant.

19. Procédé selon les revendications 1 à 18, dans lequel le contrôle des quantités d'amorces à but diagnostique ou d'amorces à but diagnostique chimère allongées comprend la quantification de séquences d'acide nucléique cible, en particulier le génotypage d'échantillons de séquences d'acide nucléique cible, de préférence (i) en calculant le rapport des valeurs de signaux enregistrés individuellement, ou (ii) en soustrayant la valeur du signal B de la valeur du signal A, ou (iii) en divisant la valeur du reste du signal A, après soustraction du signal B, par la valeur de la somme du signal A et du signal B.

20. Utilisation du procédé selon les revendications 1 à 19 pour la détection de la présence ou de l'absence d'un ou plusieurs nucléotides variants dans un échantillon de séquences d'acide nucléique cible.

21. Utilisation du procédé selon la revendication 20 pour le criblage diagnostique d'un échantillon de séquences d'acide nucléique cible pour identifier des états et des maladies héréditaires ou acquis et pour le criblage de prédispositions à de tels états et maladies.

22. Utilisation du procédé selon les revendications 1 à 19 pour la quantification de séquences d'acide nucléique cible, en particulier pour le génotypage de séquences d'acide nucléique cible, la détermination de la concentration d'ADN, d'ARN, d'ARNm ou d'ARN viral dans un échantillon, ou la détermination du nombre de copies de séquences d'acide nucléique cible, en particulier la détermination du nombre de copies de séquences d'acide nucléique cible dans des échantillons provenant de tissu tumoral.

23. Utilisation du procédé selon les revendications 1 à 19 pour le dosage de l'état de méthylation, de préférence l'état de méthylation de la cytosine, de séquences d'acide nucléique cible.

24. Utilisation d'une trousse pour la détection de la présence ou de l'absence de nucléotides variants dans un échantillon de séquences d'acide nucléique cible dans un procédé selon les revendications 1 à 19, ladite trousse comprenant, en combinaison encapsidée :

- des nucléoside-triphosphates ou des dérivés fonctionnels de ceux-ci
- un agent polymérisant
- au moins une paire d'amorces d'amplification spécifiques pour une cible capables de s'hybrider sur des séquences d'acide nucléique cible
- au moins un jeu d'amorces à but diagnostique, chaque jeu étant constitué d'au moins deux types d'amorces à but diagnostique capables de s'hybrider de manière spécifique pour un allèle variable sur des séquences d'acide nucléique cible correspondantes côté 3' par rapport auxdites amorces d'amplification, de sorte que chaque jeu d'amorces à but diagnostique est semi-niché par rapport à la paire correspondante d'amorces d'amplification,
les au moins deux types d'amorces à but diagnostique d'un jeu d'amorces à but diagnostique étant **caractérisés en ce qu'**ils ont des séquences de nucléotides essentiellement similaires à l'exception d'au moins un nucléotide interne, qui est différent pour chaque type d'amorce à but diagnostique et complémentaire du nucléotide variant, si bien qu'une amorce à but diagnostique allongée est synthétisée si le nucléotide interne de l'amorce à but diagnostique est complémentaire du nucléotide correspondant dans la séquence d'acide nucléique cible, et si bien qu'un bruit de fond essentiellement nul ou négligeable d'amorce à but diagnostique allongée est synthétisé si ledit nucléotide interne n'est pas complémentaire du nucléotide correspondant dans la séquence d'acide nucléique cible,
et chaque type d'amorce à but diagnostique étant en outre **caractérisé en ce qu'**il porte un marqueur différent et pouvant être différencié.

25. Utilisation d'une trousse selon la revendication 24, dans laquelle ladite amorce à but diagnostique est une amorce à but diagnostique chimère.

26. Utilisation d'une trousse selon la revendication 25, comprenant en outre des sondes spécifiques pour une queue 5'.

27. Utilisation d'une trousse selon les revendications 24 à 26, comprenant au moins un échantillon de séquences d'acide nucléique, en tant qu'étalon interne.

28. Utilisation d'une trousse selon les revendications 24 à 27 pour la détection de la présence ou de l'absence d'un ou plusieurs nucléotides variant associés à des états ou des maladies héréditaires ou acquis, pour le génotypage, pour la quantification de séquences d'acide nucléique cible et pour le dosage de l'état de méthylation, de préférence l'état de méthylation de la cytosine de séquences d'acide nucléique cible.

# Figure 1

**Allele A**

**Allele B**

# Figure 2

# Figure 3

A

| | | |
|---|---|---|
| PCR product | 191 bp | |
| wildtype allele | 153 bp | Cla I  38 bp |
| mutant allele | 191 bp | |

B

600 bp →

191 bp →
153 bp →

C

D

| | (543-633)/(543+633) mean ± SD | CI 99.99 % |
|---|---|---|
| wt / wt | 0.94 ± 0.04 | 0.83 - 1.00 |
| wt / mut | - 0.18 ± 0.01 | -0.16 - -0.20 |
| mut / mut | -0.98 ± 0.002 | -0.97 - -0.99 |

# Figure 4

A

B

| 3x3 sec | (543-633)/(543+633)<br>mean ± SD | CI 99.99 % |
|---|---|---|
| wt / wt | 0.95 ± 0.035 | 0.93 - 0.97 |
| wt / mut | - 0.08 ± 0.14 | -0.01 - -0.15 |
| mut / mut | -0.96 ± 0.05 | -0.94 - -0.98 |

C

| 1x3 sec | (543-633)/(543+633)<br>mean ± SD | CI 99.99 % |
|---|---|---|
| wt / wt | 0.97 ± 0.06 | 0.94 - 1.00 |
| wt / mut | -0.06 ± 0.16 | 0.01 - -0.13 |
| mut / mut | -0.95 ± 0.08 | -0.91 - -0.99 |

# Figure 5

A

B

# Figure 6

# Figure 7

# Figure 8

A

B

372 bp →

C

| | (543-633)/(543+633) mean ± SD | CI 99.99 % |
|---|---|---|
| wt / wt | 0.95 ± 0.06 | 0.89 – 1.00 |
| wt / mut | 0.05 ± 0.08 | -0.02 - 0.12 |
| mut / mut | -0.87 ± 0.05 | -0.82 - -0.92 |

# Figure 9

**EP 1 474 529 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 332435 A
- WO 0204686 A
- EP 0301276 W

**Non-patent literature cited in the description**

- **COOPER et al.** *Hum Genet,* 1985, vol. 69, 201-205
- **COLLINS et al.** *Genome Res,* 1998, vol. 8, 1229-1231
- **LANDER et al.** *Nature,* 2001, vol. 409, 860-921
- **VENTER et al.** *Science,* 2001, vol. 291, 1304-1351
- **BENTLEY.** *Med Res Rev,* 2000, vol. 20, 189-196
- **PFOST et al.** *Trends Biotechnol,* 2000, vol. 18, 334-338
- **SCHORK et al.** *Clin Genet,* 2000, vol. 58, 250-264
- **NICKERSON et al.** *Proc Natl Acad Sci USA,* 1990, vol. 87, 8923-8927
- **SAIKI et al.** *Proc Natl Acad- Sci USA,* 1989, vol. 86, 6230-6234
- **DE VERLAAN et al.** *Gene,* 1986, vol. 50, 313-320
- **WALLACE et al.** *Nucleic Acids Res,* 1981, vol. 9, 879-894
- **ZHANG et al.** *Nucleic Acids Res,* 1991, vol. 19, 3929-3933
- **GIBBS et al.** *Nucleic Acids Res,* 1989, vol. 17, 2437-2448
- **NEWTON et al.** *Nucleic Acids Res,* 1989, vol. 17, 2503-2516
- **GROSSMAN et al.** *Nucleic Acids Res,* 1994, vol. 22, 4527-4534
- **LANDEGREN et al.** *Science,* 1988, vol. 241, 1077-1080
- **ABRAVAYA et al.** *Nucleic Acids Res,* 1995, vol. 23, 675-682
- **BARANY.** *Proc Natl Acad Sci USA,* 1991, vol. 88, 189-193
- **WU ; WALLACE.** *Genomics,* 1989, vol. 4, 560-569
- **WANG et al.** *Science,* 1998, vol. 280, 1077-1082
- **YERSHOV et al.** *Proc Natl Acad Sci USA,* 1996, vol. 93, 4913-4918
- **HOLLAND et al.** *Proc Natl Acad Sci USA,* 1991, vol. 88, 7276-7280
- **LEE et al.** *Biotechniques,* 1999, vol. 27, 342-349
- **LIVAK.** *Genet Anal,* 1999, vol. 14, 143-149
- **LIVAK et al.** *PCR Methods Appl,* 1995, vol. 4, 357-362
- **CHEN ; KWOK.** *Nucleic Acids Res,* 1997, vol. 25, 347-353
- **CHEN et al.** *Genome Res,* 1997, vol. 9, 492-498
- *Proc Natl. Acad Sci USA,* 1997, vol. 94, 10756-10761
- **MARRAS et al.** *Genet Anal,* 1999, vol. 14, 151-156
- **TYAGI ; KRAMER.** *Nat Biotechnol,* 1996, vol. 14, 303-308
- **BERNARD et al.** *Anal Biochem,* 1998, vol. 255, 101-107
- **LAY ; WITTWER.** *Clin Chem,* 1997, vol. 43, 2262-2267
- **LYAMICHEV et al.** *Nat Biotechnol,* 1999, vol. 17, 292-296
- **AHMADIAN et al.** *Anal Biochem,* 2000, vol. 280, 103-110
- **ALDERBORN et al.** *Genome Res,* 2000, vol. 10, 1249-1258
- **KWOK S. et al.** *Nucleic Acids Research,* 1990, vol. 18 (4), 999-1005
- *Acad Sci USA,* 1994, vol. 91, 5740-5747
- **ELSON ; MAGDE.** *Biopolymers,* 1974, vol. 13, 1-27
- **MAGDE et al.** *Phys Rev Lett,* 1972, vol. 29, 705-708
- *Biopolymers,* 1974, vol. 13, 29-61
- **AUER et al.** *Drug Discovery Today,* 1998, vol. 3, 457-465
- **STERRER ; HENCO.** *J Recept Signal Transduct Res,* 1997, vol. 17, 511-520
- **KETTLING et al.** *Proc Natl Acad Sci USA,* 1998, vol. 95, 1416-1420
- **MEYER-ALMES ; AUER.** *Biochemistry,* 2000, vol. 39, 13261-13268

- **PITSCHKE et al.** *Nat Med,* 1998, vol. 4, 832-834
- **TJERNBERG et al.** *Chem Biol,* 1999, vol. 6, 53-62
- **BJORLING et al.** *Biochemistry,* 1998, vol. 37, 12971-12978
- **KINJO.** *BioTechniques,* 1998, vol. 25, 705-6
- **KINJO et al.** *Anal Biochem,* 1998, vol. 260, 166-172
- **KINJO ; RIGLER.** *Nucleic Acids Res,* 1995, vol. 23, 1795-1799
- **RIGLER et al.** *J Biotechnol,* 1998, vol. 63, 97-109
- **WALTER et al.** *Proc Natl Acad Sci USA,* 1996, vol. 93, 12805-12810
- **WEINER et al.** *Digestion,* 2000, vol. 61, 84-89
- **KINJO ; NISHIMURA.** *Bioimaging,* 1997, vol. 5, 134-138
- **NEWTON et al.** *Nucleic Acid Res,* 1989, vol. 17, 2503-16
- **BOLDT et al.** *Brit J Haemat,* 1997, vol. 99, 968-973
- **HAMAJIMA et al.** *Jpn J Cancer Res,* 2000, vol. 91, 865-868
- **HAFF.** *PCR Methods Appl,* 1994, vol. 3, 332-337
- **GUSCHIN et al.** *Anal. Biochem.,* 1997, vol. 250, 203-211
- **BLANCHARD et al.** *Biosens. Bioelectron.,* 1996, vol. 11, 687-690
- **FODOR et al.** *Science,* 1991, vol. 251, 767-773
- **MCGALL et al.** *Proc. Natl. Acad. Sci. USA,* 1996, vol. 93, 13555-13560
- **VASILISKOV et al.** *BioTechniques,* 1999, vol. 27, 592-606
- **LAWYER et al.** *J Biol Chem,* 1989, vol. 264, 6427-6437
- **LAWYER et al.** *PCR Method Appl,* 1993, vol. 2, 275-287
- **SAMBROOK ; RUSSELL.** Molecular Cloning: A Laboratory Manual. Cold Spring Harbor Laboratory Press, 2000
- **BIRD.** *Cell,* 1992, vol. 70, 5-8
- **LI et al.** *Nature,* 1993, vol. 366, 362-365
- **PFEIFER et al.** *Science,* 1989, vol. 246, 810-813
- **ANTEQUERA et al.** *Cell,* 1990, vol. 62, 503-514
- **HERMAN et al.** *Proc Natl Acad Sci USA,* 1996, vol. 93, 9821-9826
- **WANG et al.** *Nucleic Acids Res,* 1980, vol. 8, 4777-4790
- **PAPADOPOULOS et al.** *Science,* 1994, vol. 263, 1625-1629
- **LIU et al.** *Nat Genet,* 1995, vol. 9, 48-55
- **TANNERGARD et al.** *Cancer Res,* 1995, vol. 55, 6092-6096
- **LIU et al.** *Nat Med,* 1996, vol. 2, 169-174
- **SCHWILLE et al.** *Biophys J,* 1997, vol. 72, 1878-1886